# EUROPEAN PATENT APPLICATION

(11) **EP 4 835 458 A1**
(43) Date of publication of application: **04.11.2026**
(21) Application number: 25834105.6
(22) Date of filing: 12.03.2025
(51) Int. Cl.: G01N 24/08

(54) **NUCLEAR MAGNETIC RESONANCE IDENTIFICATION METHOD FOR HAINA POLYSACCHARIDE**

(30) Priority: 06.03.2025 CN 202510261309
(71) Applicant: Harbin Hongdoushan Bio-pharm Co., Ltd., Harbin, Heilongjiang 150600 (CN)
(72) Inventor: HAN, Li, Harbin, Heilongjiang 150600 (CN); WANG, Yicheng, Harbin, Heilongjiang 150600 (CN); HUANG, Xueshi, Harbin, Heilongjiang 150600 (CN); WANG, Zhiguo, Harbin, Heilongjiang 150600 (CN); MU, Yu, Harbin, Heilongjiang 150600 (CN); WU, Xuehai, Harbin, Heilongjiang 150600 (CN); MENG, Yiqiang, Harbin, Heilongjiang 150600 (CN)
(74) Representative: LEITZINGER OY
(86) International application number: PCT/CN2025/082090
(87) International publication number: WO 2026/183871

(57) **Abstract**

An identification method of a Haina polysaccharide (HPS) by nuclear magnetic resonance (NMR) spectroscopy is provided, relating to the technical field of pharmaceuticals. The identification method includes: mixing a sample to be tested, an internal standard, and deuterium oxide to obtain a test solution; subjecting the test solution to NMR spectroscopy test, and analyzing a resulting hydrogen nuclear magnetic resonance (¹H NMR) spectrum; and identifying whether the sample to be tested is a qualified product of the HPS based on an obtained analysis result; where the NMR spectroscopy test is conducted under conditions including: a frequency not less than 600 MHz, a pulse angle of 90°, an acquisition time not less than 2 s, a relaxation delay not less than 12 s, a number of scans not less than 16, and a spectral width covering at least a range from 10 ppm to 0 ppm. The identification method establishes, for the first time, an identification approach for the HPS using NMR spectroscopy, which is simple in operations and highly specific, thereby enabling effective identification of the HPS.

## Description

### TECHNICAL FIELD

The present disclosure relates to the technical field of pharmaceuticals, and in particular to an identification method of a Haina polysaccharide (HPS) by nuclear magnetic resonance (NMR) spectroscopy.

### BACKGROUND

Sea cucumbers in their body wall are rich in polysaccharides, primarily including two major categories: fucoidan and fucosylated chondroitin sulfate. Among these, sea cucumber-derived fucosylated chondroitin sulfate demonstrates a wide range of biological activities due to its unique structure, such as lipid-lowering, antitumor, antiviral, and anti-inflammatory effects. Notably, the fucosylated chondroitin sulfate possesses excellent anticoagulant and antithrombotic activities.

Currently, pharmacopoeial identification methods of polysaccharide drugs are relatively limited. They generally include total sugar content determination, monosaccharide composition analysis by thin-layer chromatography, or infrared identification. However, these methods lack specificity. Presently, an identification method suitable for Haina polysaccharide (HPS) has not been established in the field.

### SUMMARY

In view of this, the present disclosure provides an identification method of a Haina polysaccharide (HPS) by nuclear magnetic resonance (NMR) spectroscopy. In the present disclosure, the identification method is simple in operations and highly specific, and enables effective identification of the HPS and recognizes qualified products of the HPS.

To achieve the above objective, the present disclosure provides the following technical solutions:

The present disclosure provides an identification method of an HPS by NMR spectroscopy, including the following steps:
mixing a sample to be tested, an internal standard, and deuterium oxide to obtain a test solution;
subjecting the test solution to NMR spectroscopy test, thereby obtaining a hydrogen nuclear magnetic resonance (¹H NMR) spectrum; and
analyzing the ¹H NMR spectrum, and identifying whether the sample to be tested is a qualified product of the HPS based on an obtained analysis result; where
the NMR spectroscopy test is conducted under conditions including: a frequency not less than 600 MHz, a pulse angle of 90°, an acquisition time not less than 2 s, a relaxation delay not less than 12 s, a number of scans not less than 16, and a spectral width covering at least a range from 10 ppm to 0 ppm;
a standard for an ¹H NMR spectrum of the qualified product of the HPS comprises:
   in the ¹H NMR spectrum of the qualified product of the HPS, there are signal peaks of fucose anomeric hydrogen, N-acetyl methyl hydrogen, and fucose methyl hydrogen; relative to a methyl signal of the internal standard, a signal peak of the fucose anomeric hydrogen has a relative chemical shift of 5.68 ppm ± 0.03 ppm, a signal peak of the N-acetyl methyl hydrogen has a relative chemical shift of 2.08 ppm ± 0.03 ppm, and a signal peak of the fucose methyl hydrogen is a doublet centered at a relative chemical shift of 1.38 ppm ± 0.03 ppm;
   the signal peak of the N-acetyl methyl hydrogen in the ¹H NMR spectrum, close to a relative chemical shift of 2 ppm, has a signal-to-noise ratio not less than 2000:1;
   in relative chemical shifts of 0.2 ppm to 0.8 ppm and 6.5 ppm to 10 ppm of the ¹H NMR spectrum, no unidentified signals greater than 8% of an intensity of the signal peak of the fucose methyl hydrogen are found; in a relative chemical shift of 3 ppm to 6 ppm of the ¹H NMR spectrum, no unidentified signals greater than 120% of the intensity of the signal peak of the fucose methyl hydrogen are found; the intensity of the signal peak of the fucose methyl hydrogen is expressed as an average intensity of the doublet centered at the relative chemical shift of 1.38 ppm ± 0.03 ppm;
   an integral area ratio value of the signal peak of the N-acetyl methyl hydrogen to the signal peak of the fucose methyl hydrogen in the ¹H NMR spectrum is in a range of 1.2 to 1.8; the signal peak of the N-acetyl methyl hydrogen is integrated in a relative chemical shift range of 1.8 ppm to 2.4 ppm, and the signal peak of the fucose methyl hydrogen is integrated in a relative chemical shift range of 1.1 ppm to 1.6 ppm; and
   the HPS is a fucosylated chondroitin sulfate polysaccharide; the HPS has a weight-average molecular weight of 90,000 to 130,000, and is composed of monosaccharides including glucuronic acid, N-acetyl-D-galactosamine, and fucose, where a molar ratio of the glucuronic acid, the N-acetyl-D-galactosamine, and the fucose is in a range of 1 : 0.8-1.2 : 0.5-0.8; and a sulfate ester group in the HPS has a mass percentage of 25% to 40%.

In some embodiments, the sample to be tested in the test solution has a concentration not less than 20 mg/mL; in some embodiments, the internal standard is sodium 3-(trimethylsilyl)propionate-2,2,3,3-d4; in some embodiments, the internal standard in the test solution has a concentration of 0.002% weight/volume (w/v); in some embodiments, the NMR spectroscopy test is conducted with the acquisition time of 2 s to 3 s; in some embodiments, the NMR spectroscopy test is conducted with the relaxation delay of 12 s to 16 s; in some embodiments, the NMR spectroscopy test is conducted with the number of scans of 16 to 32; in some embodiments, the NMR spectroscopy test is conducted using an instrument mode of a pulsed Fourier transform mode; in some embodiments, the NMR spectroscopy test is conducted at a temperature of 20 °C to 30 °C.

The present disclosure provides an identification method of an HPS by NMR spectroscopy. In the present disclosure, the identification method includes: mixing a sample to be tested, an internal standard, and deuterium oxide to obtain a test solution; subjecting the test solution to NMR spectroscopy test, thereby obtaining a hydrogen nuclear magnetic resonance (¹H NMR) spectrum; and analyzing the ¹H NMR spectrum, and identifying whether the sample to be tested is a qualified product of the HPS based on an obtained analysis result. HPS is a natural fucosylated chondroitin sulfate polysaccharide obtained by the inventors from sea cucumber body wall, which can target the terminal rate-limiting enzyme iFXase in the endogenous coagulation pathway. The HPS provides excellent anticoagulant effects with minimal bleeding side effects, thereby achieving effective anticoagulation without causing bleeding. The identification method by NMR spectroscopy establishes, for the first time, an identification approach for the HPS using NMR spectroscopy, which is simple in operations and highly specific, thereby enabling effective identification of the HPS.

### BRIEF DESCRIPTION OF THE DRAWINGS

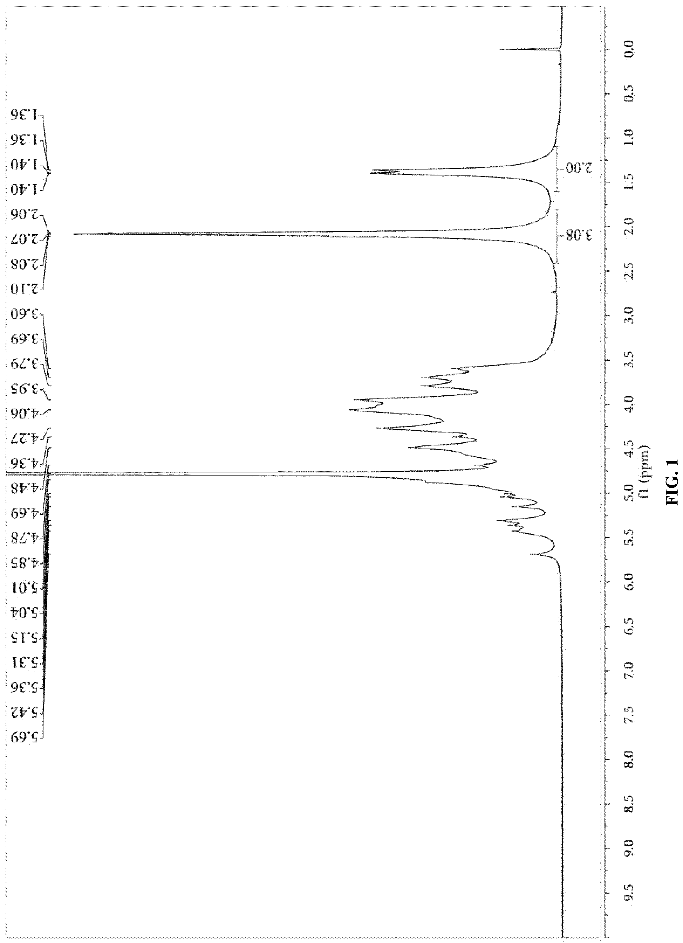

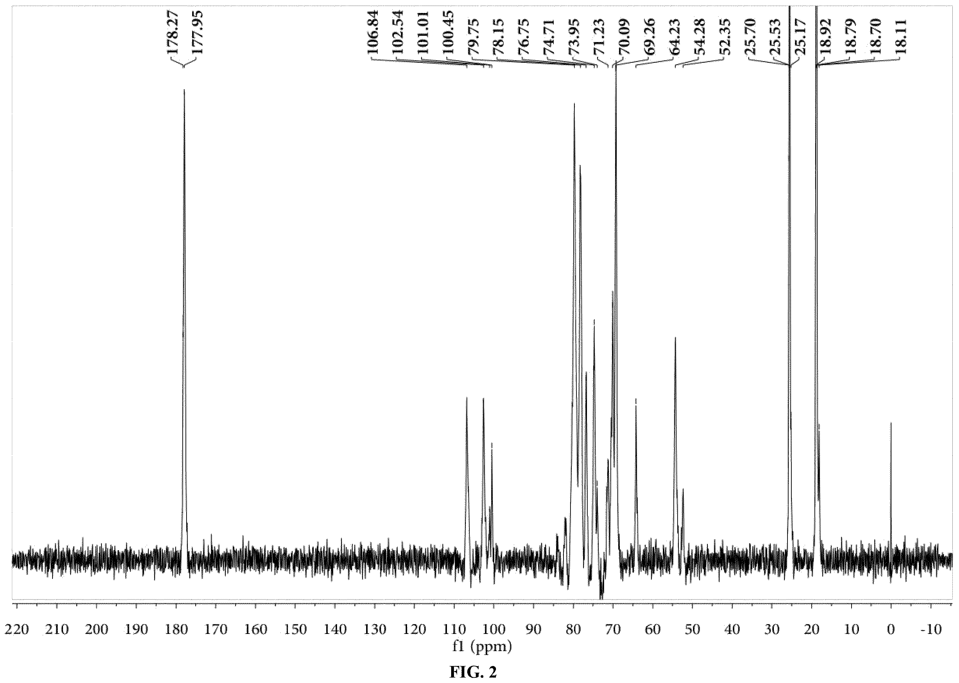

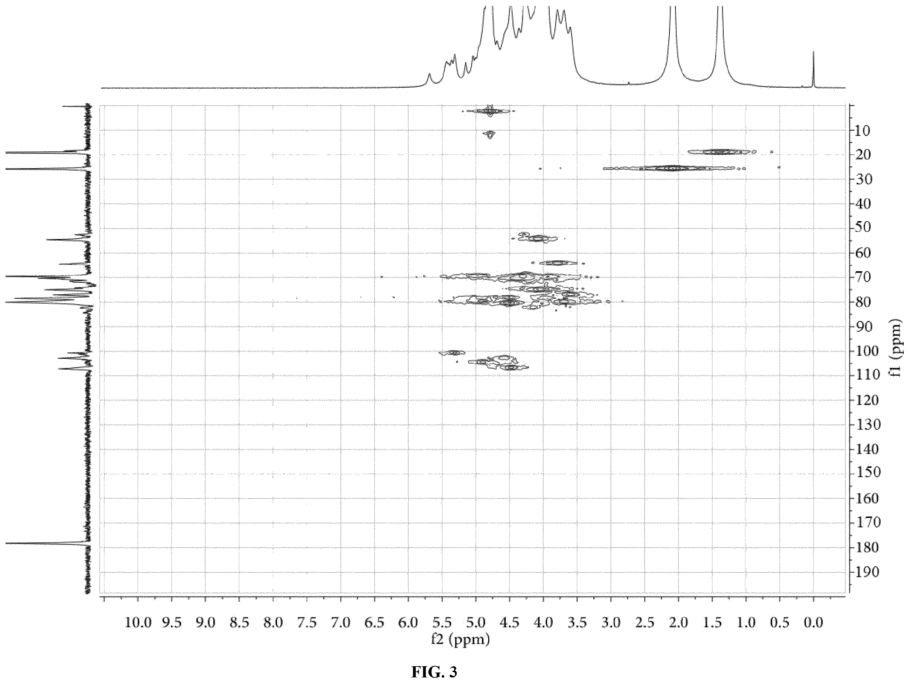

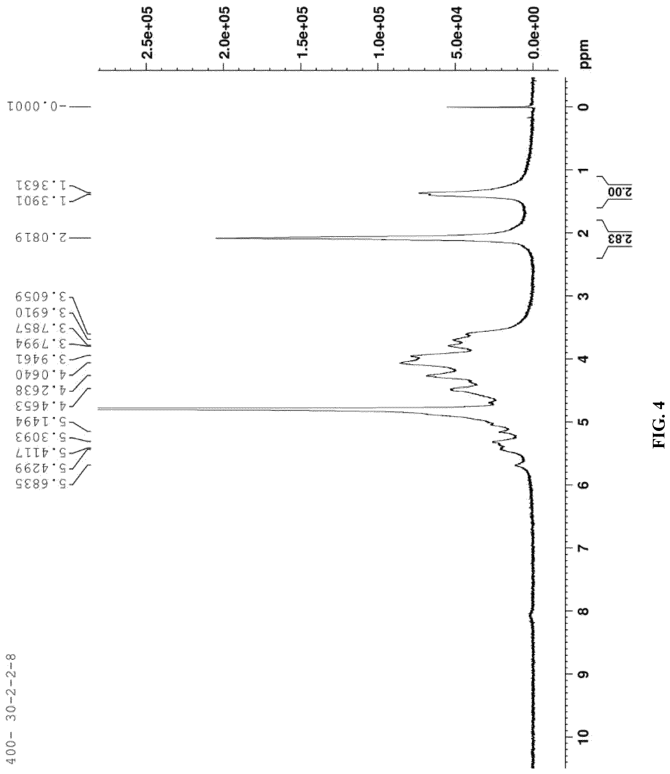

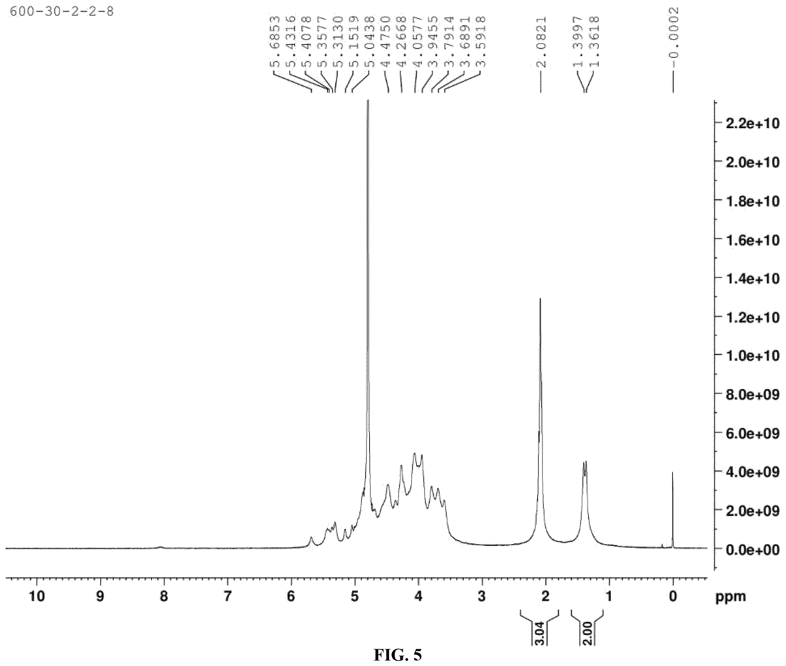

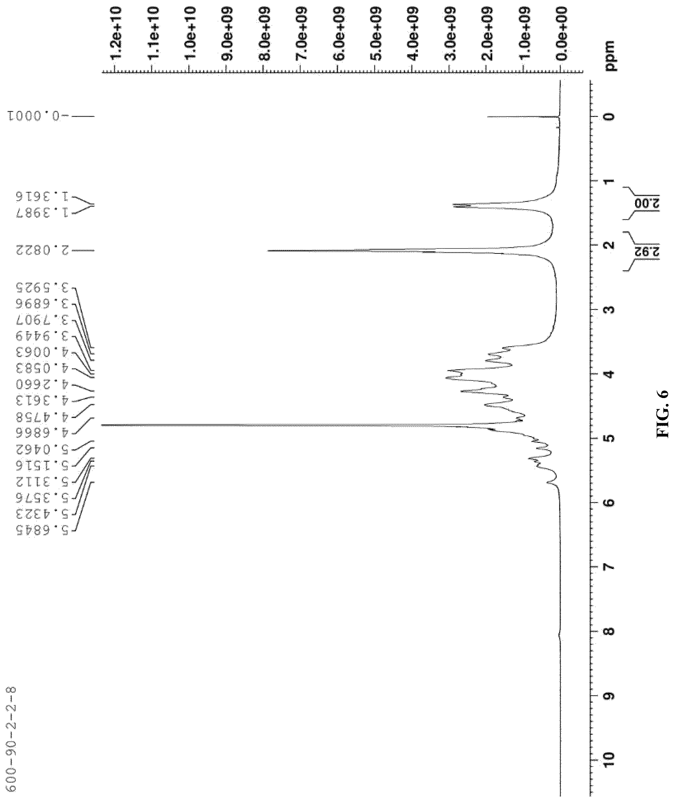

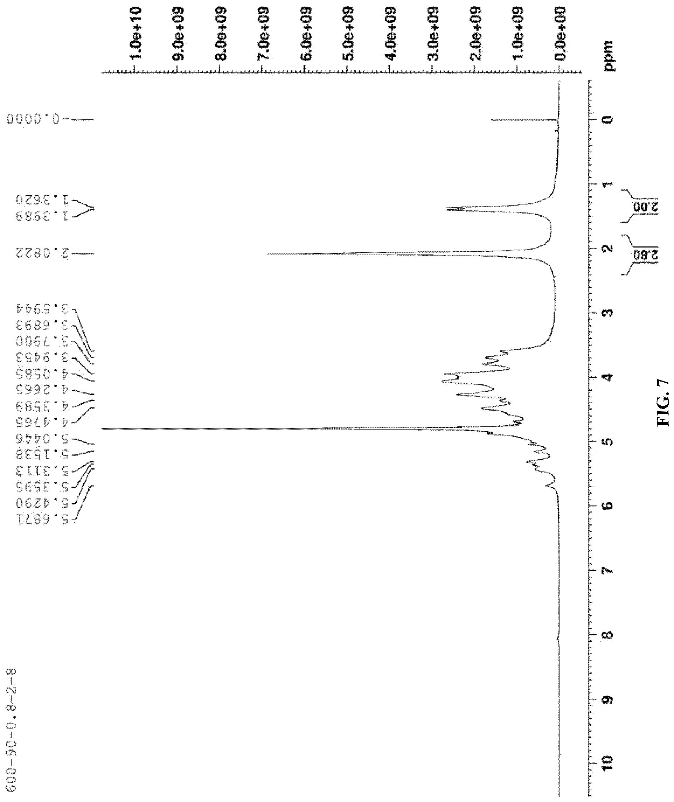

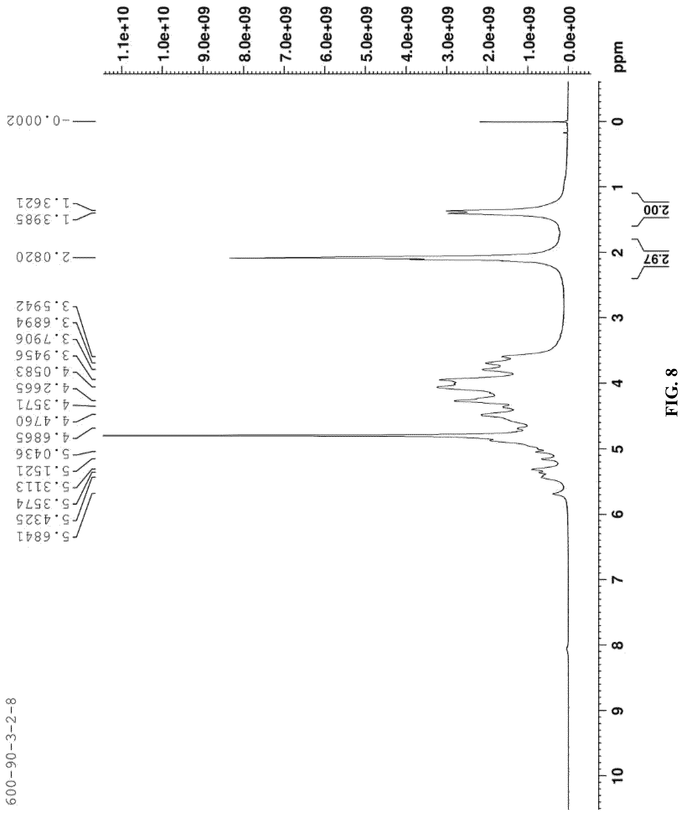

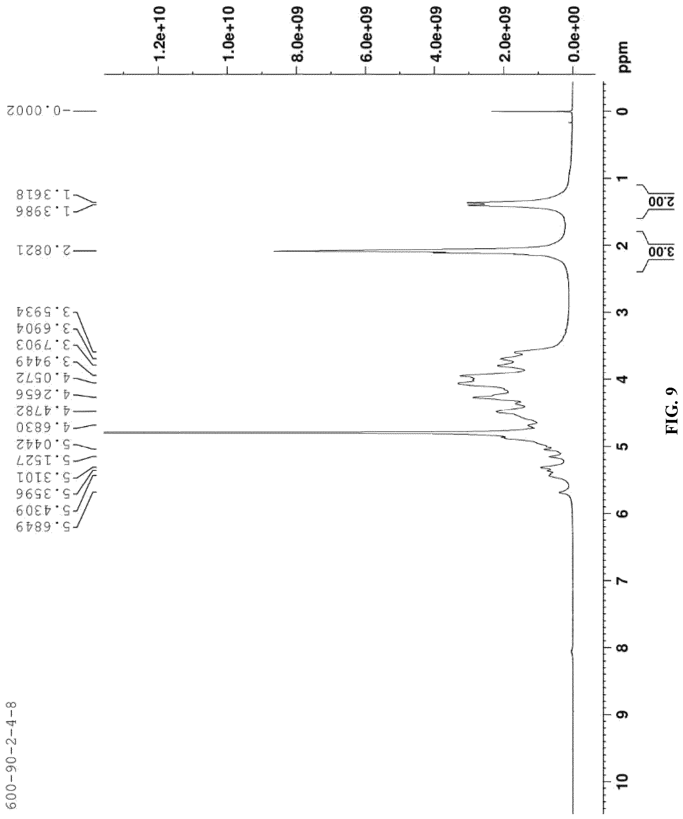

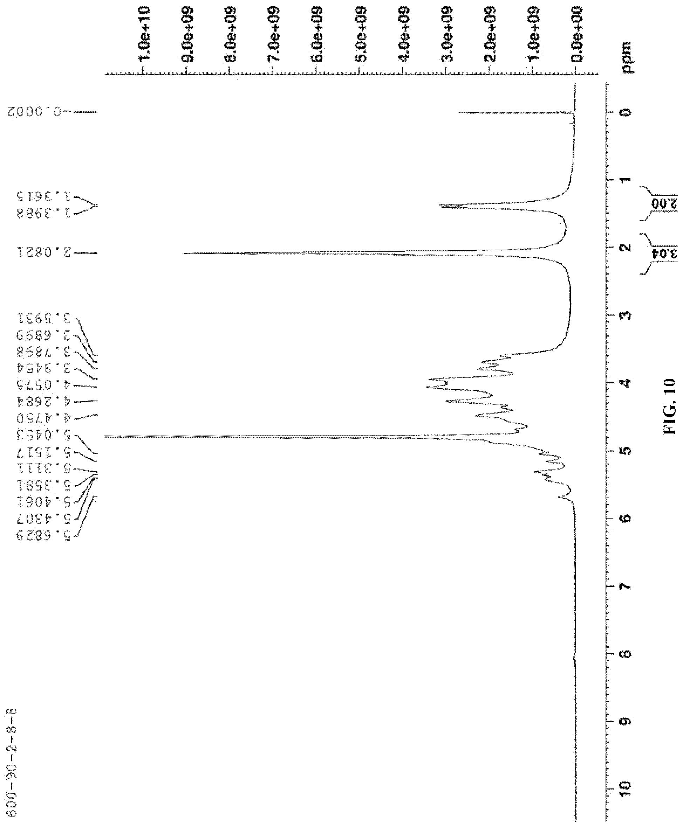

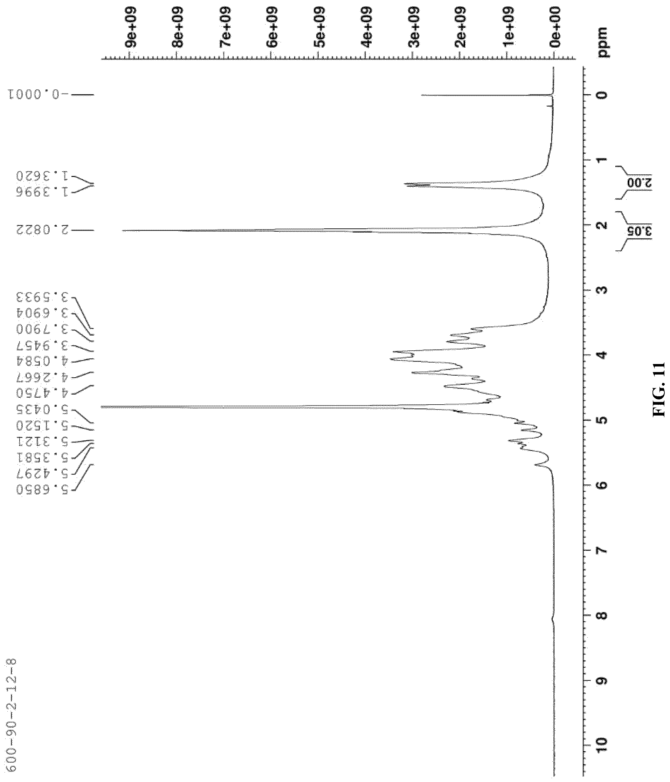

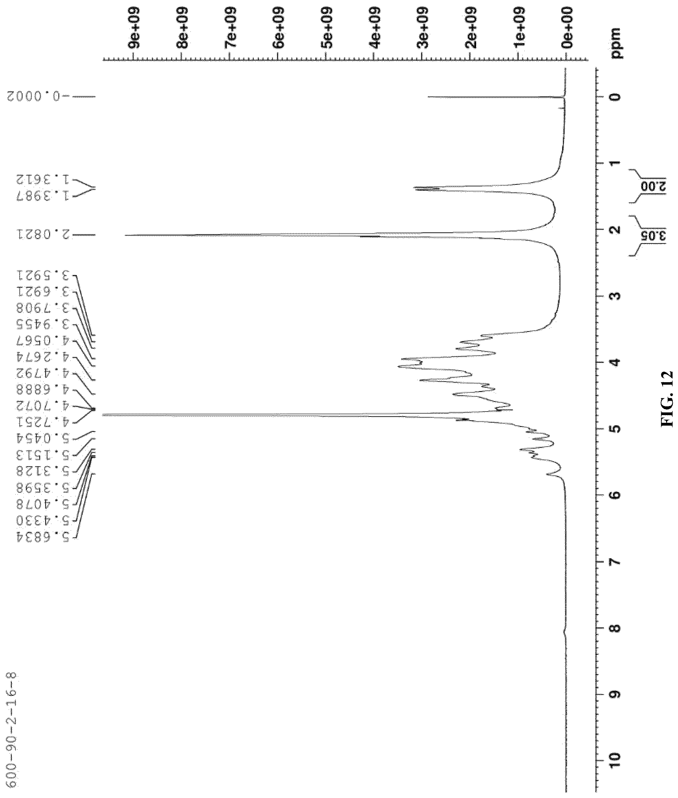

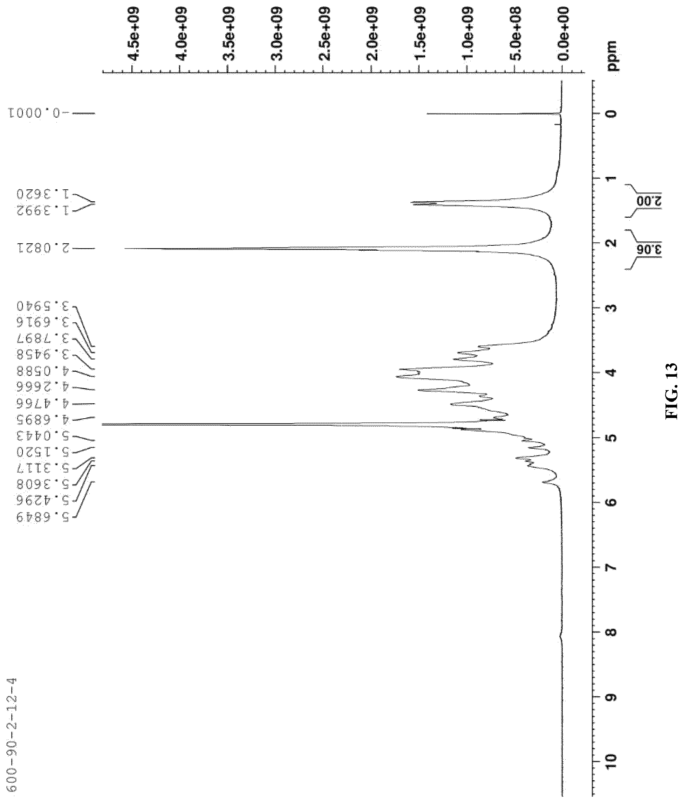

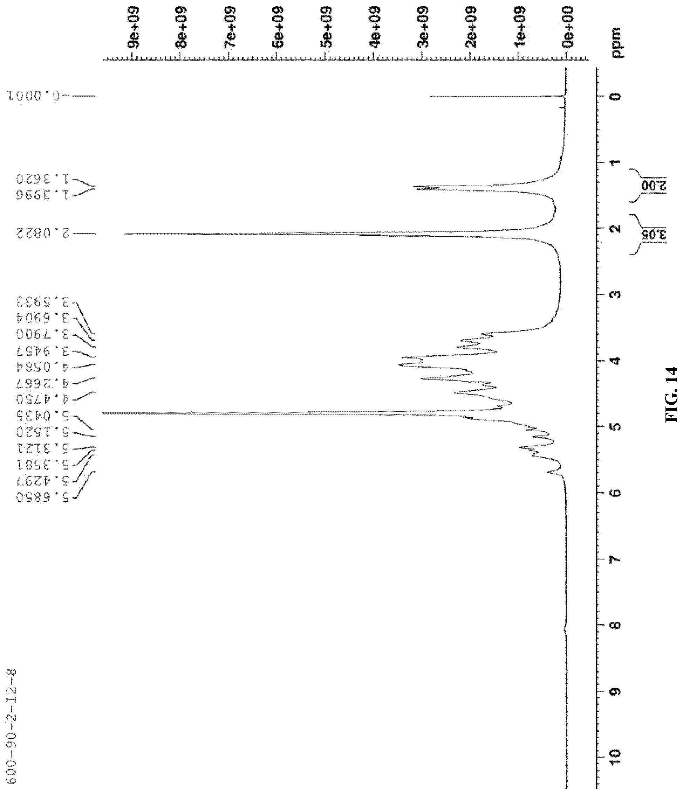

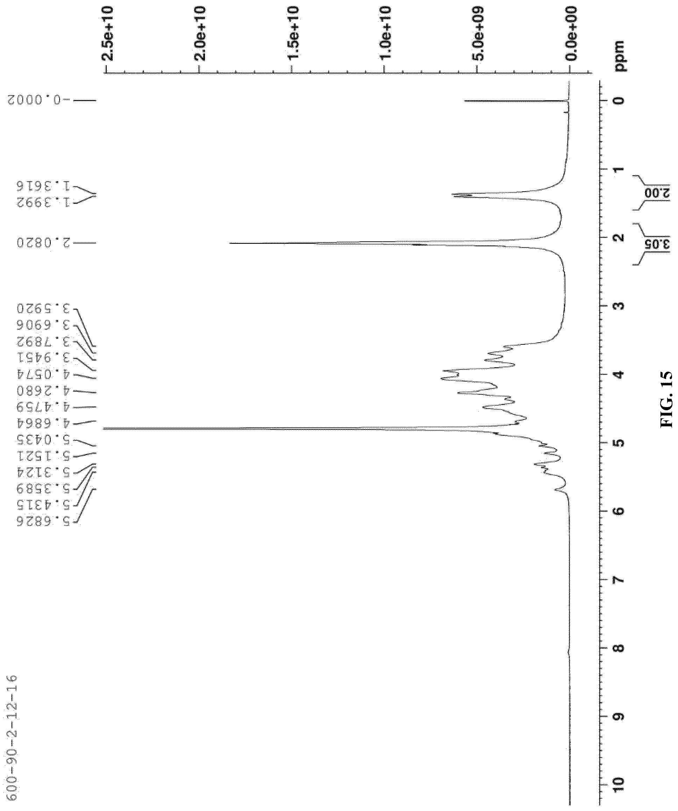

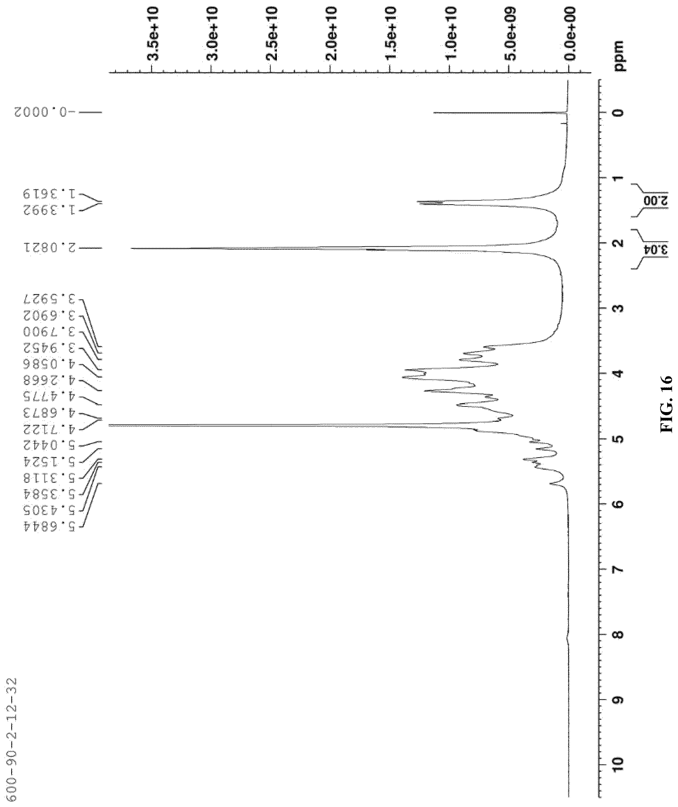

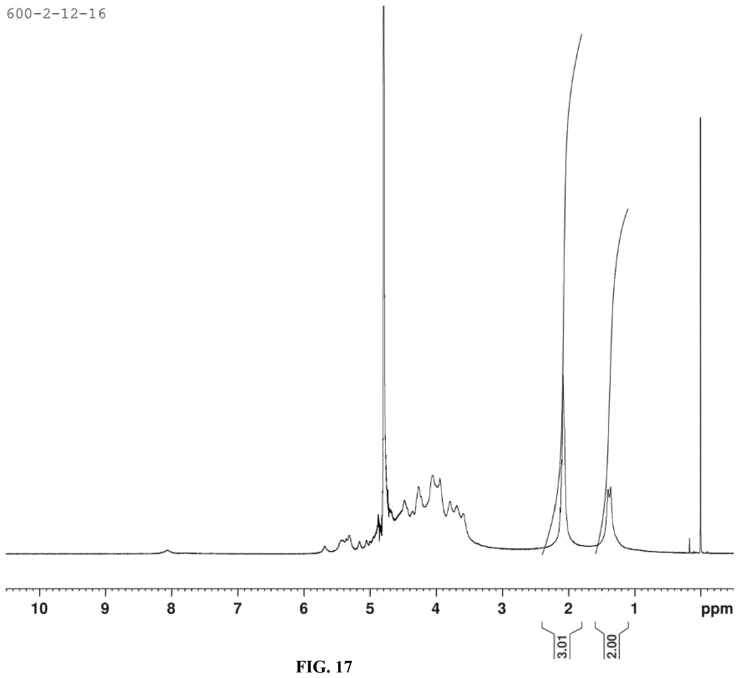

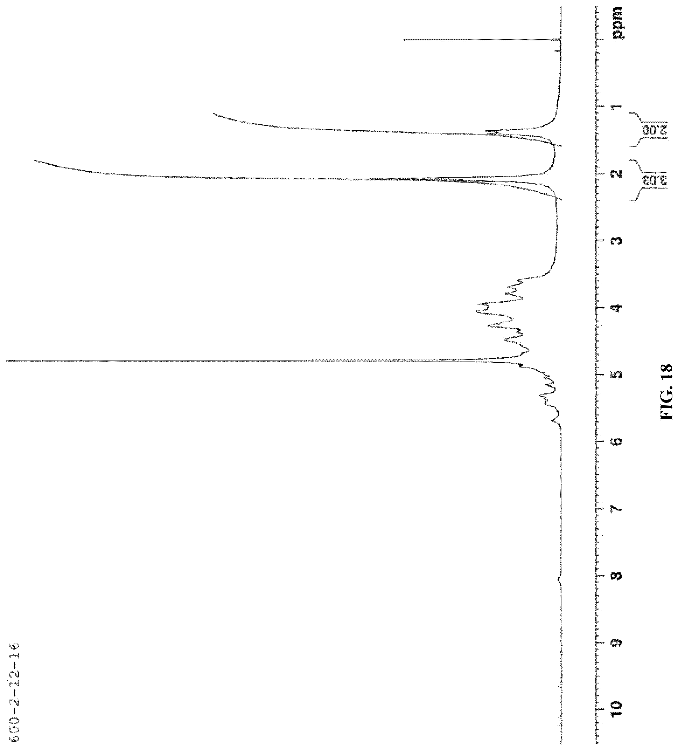

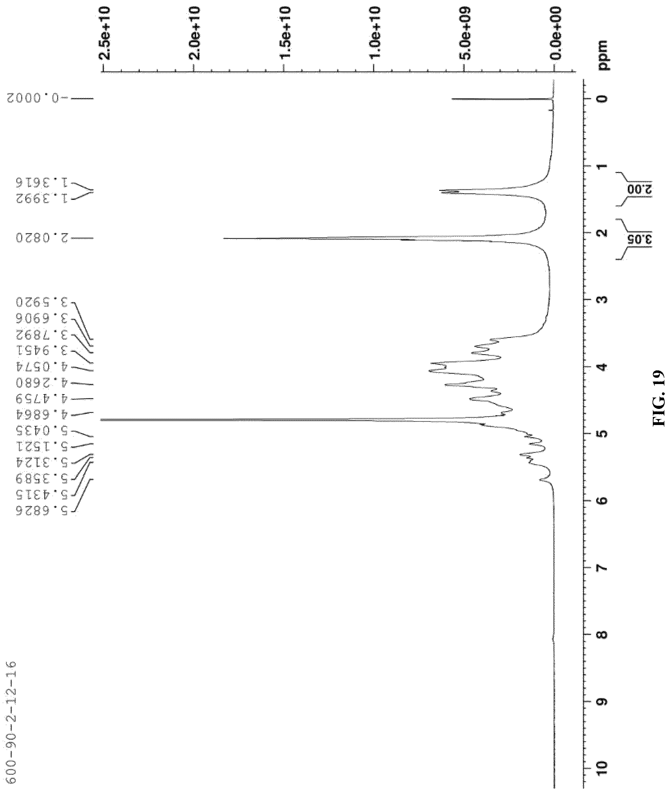

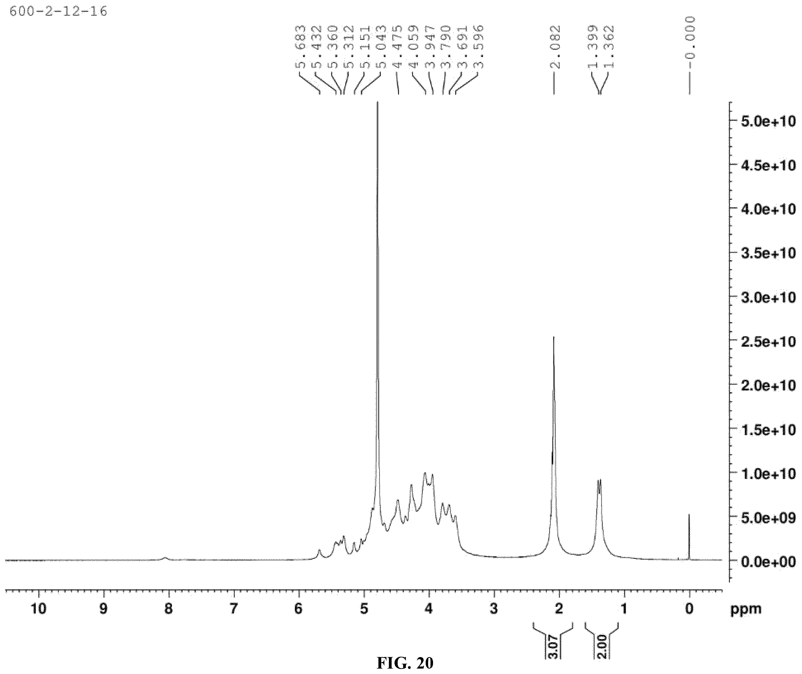

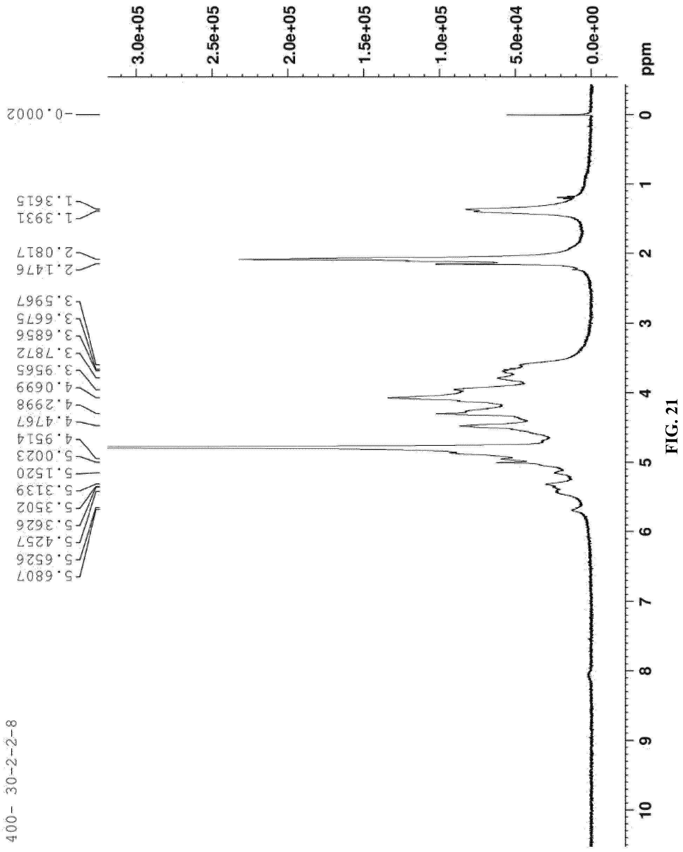

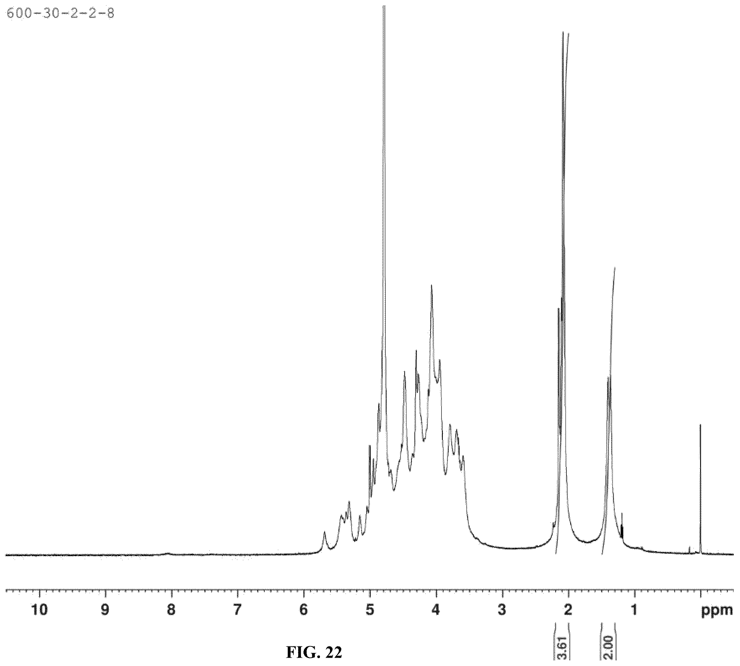

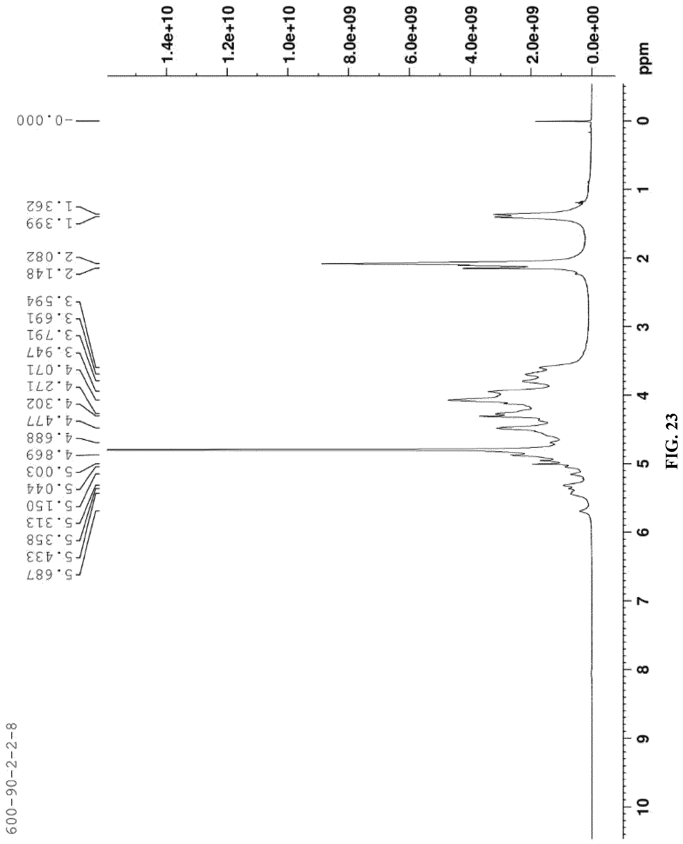

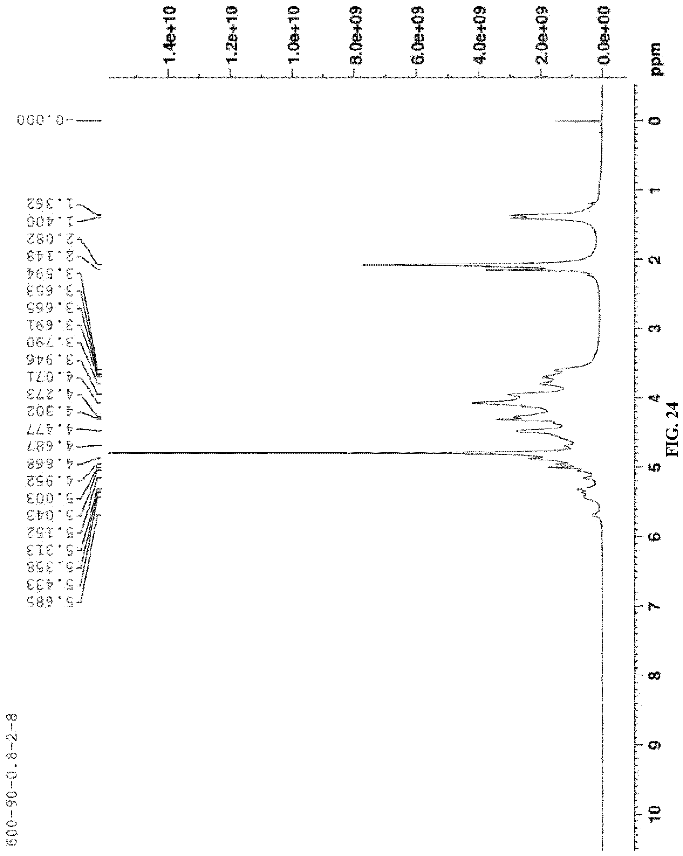

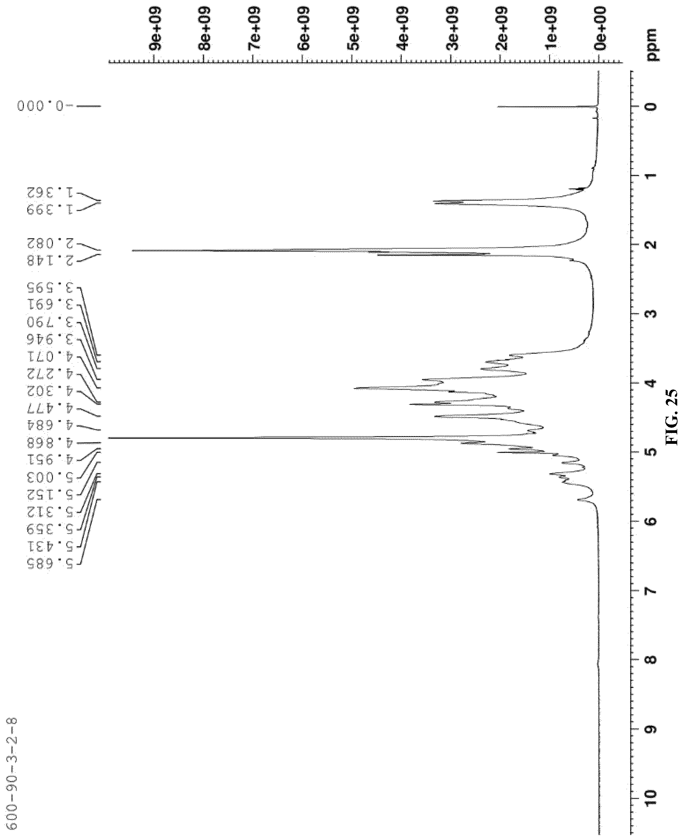

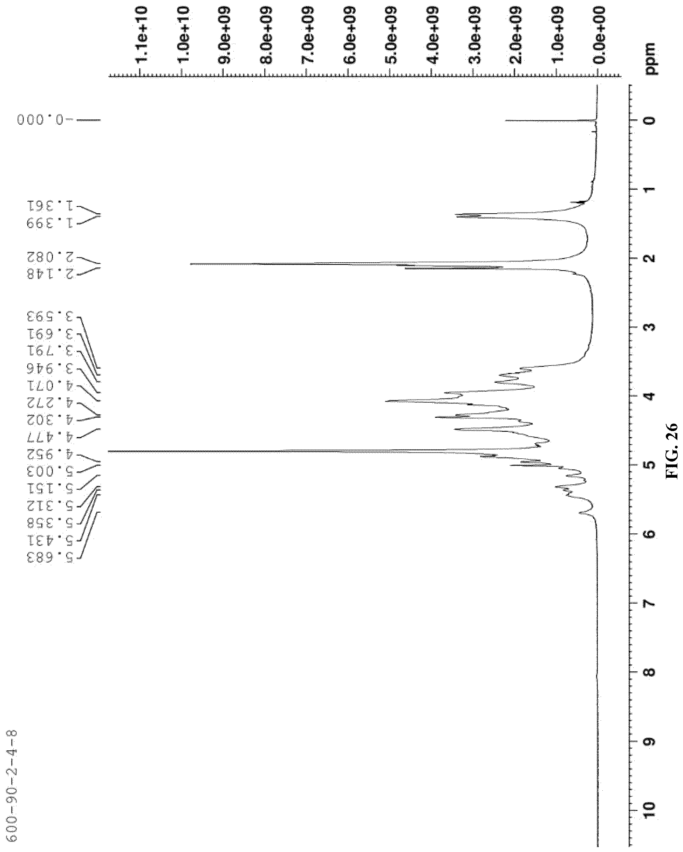

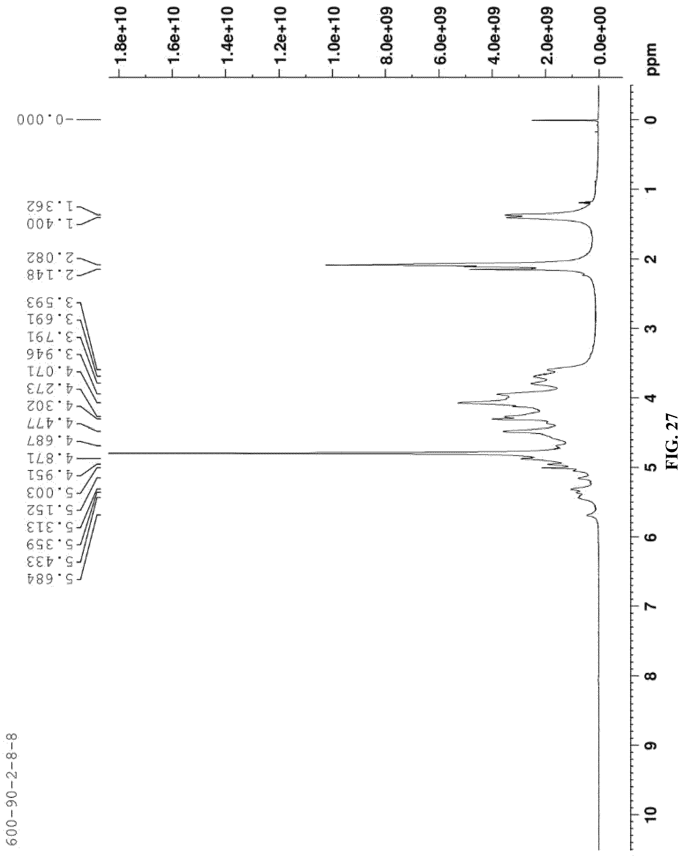

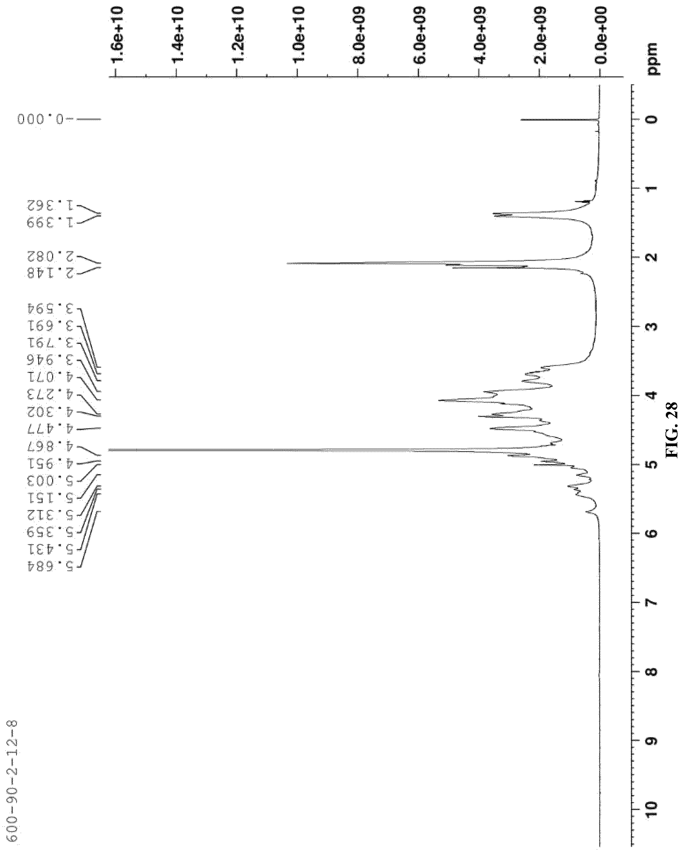

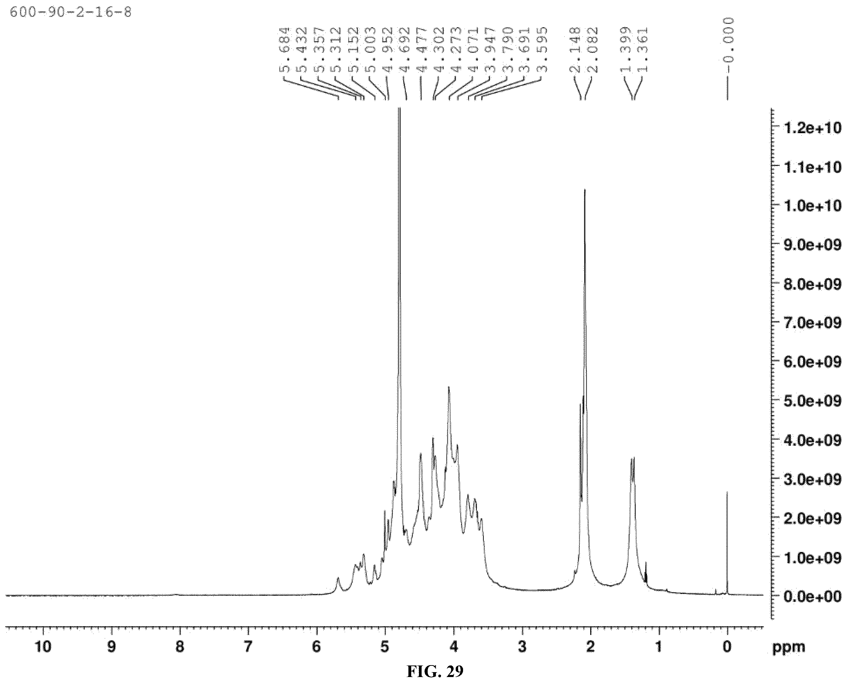

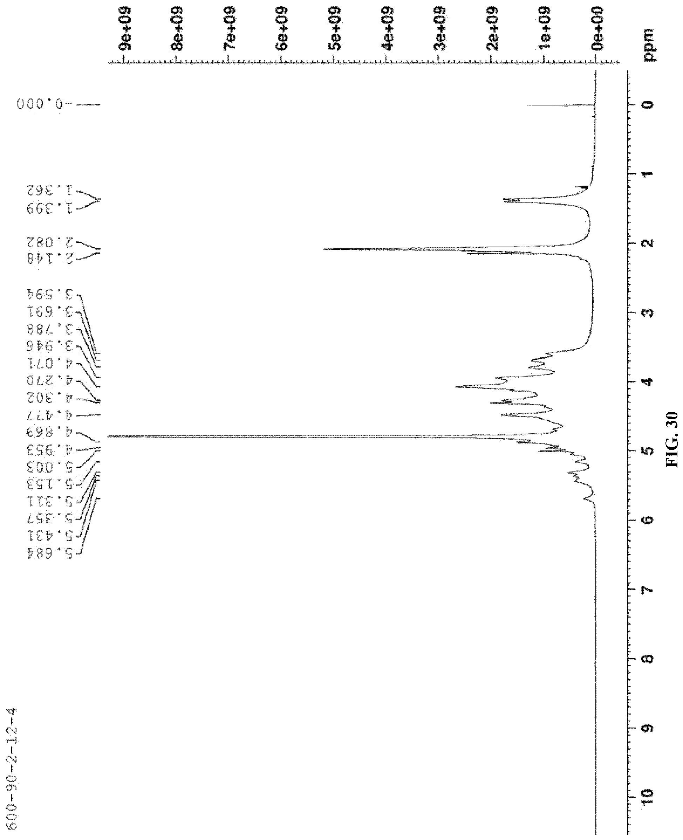

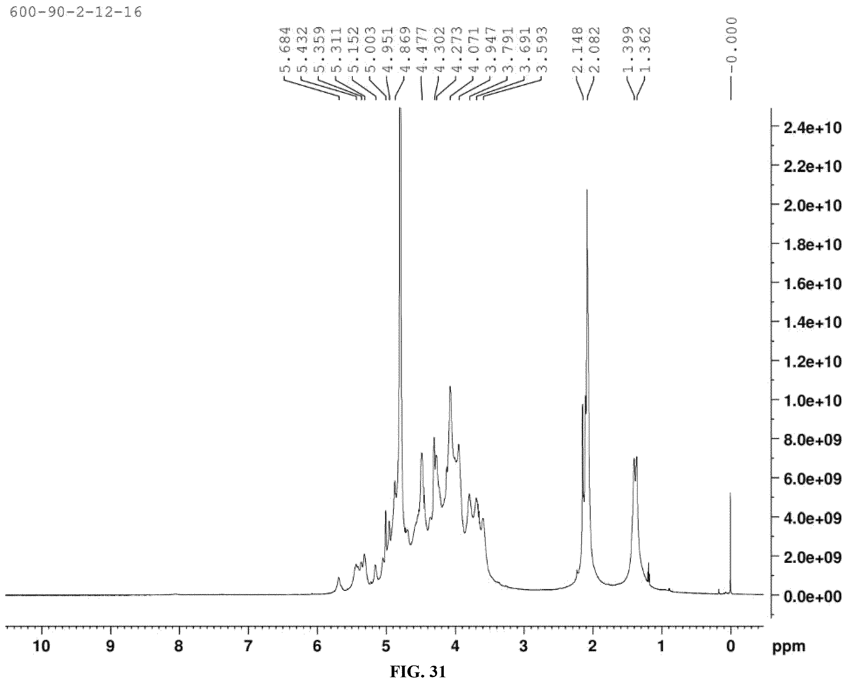

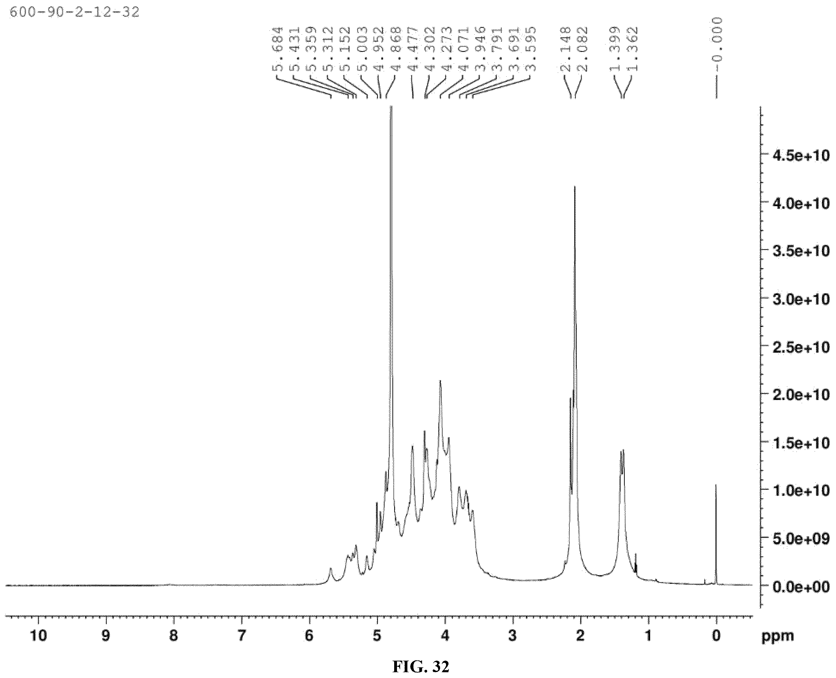

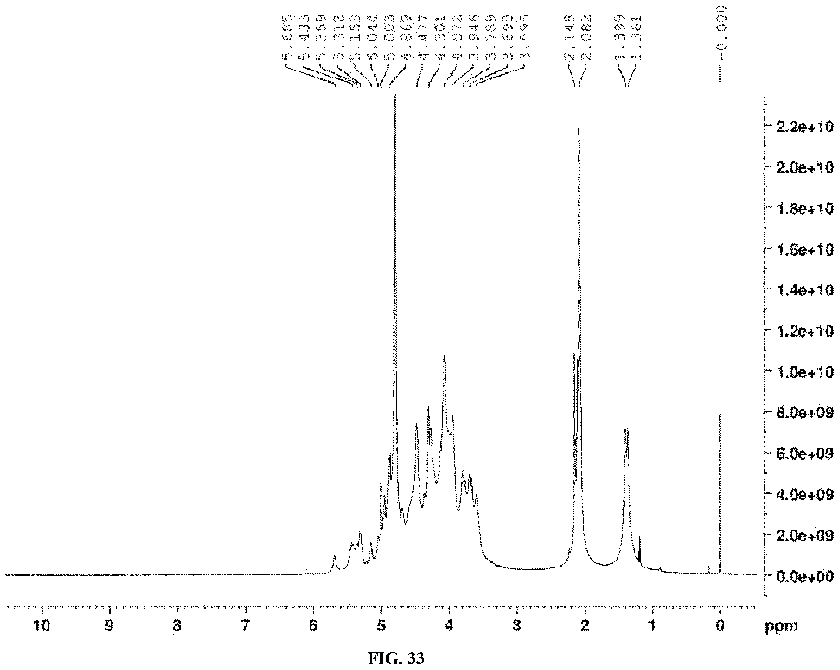

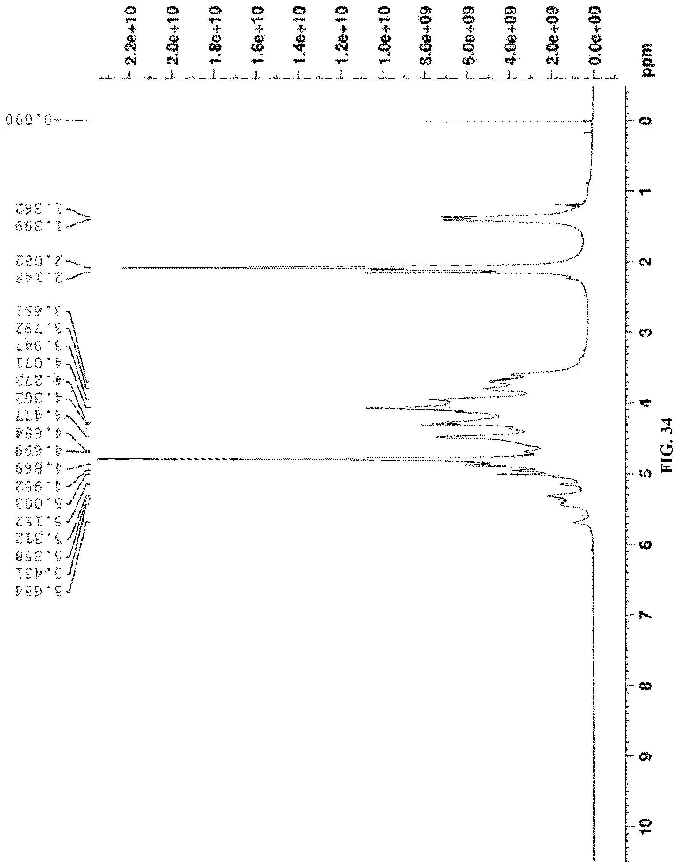

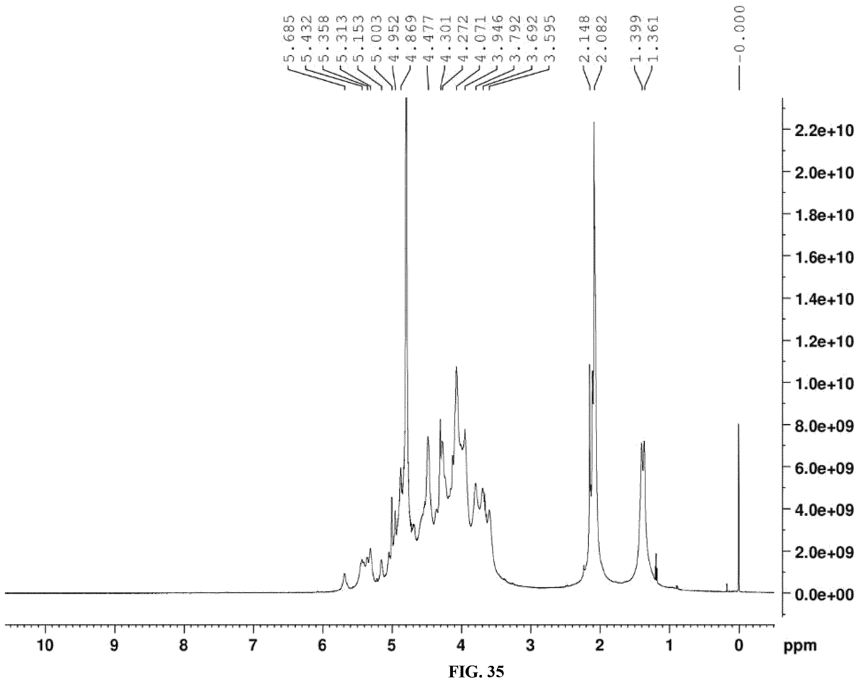

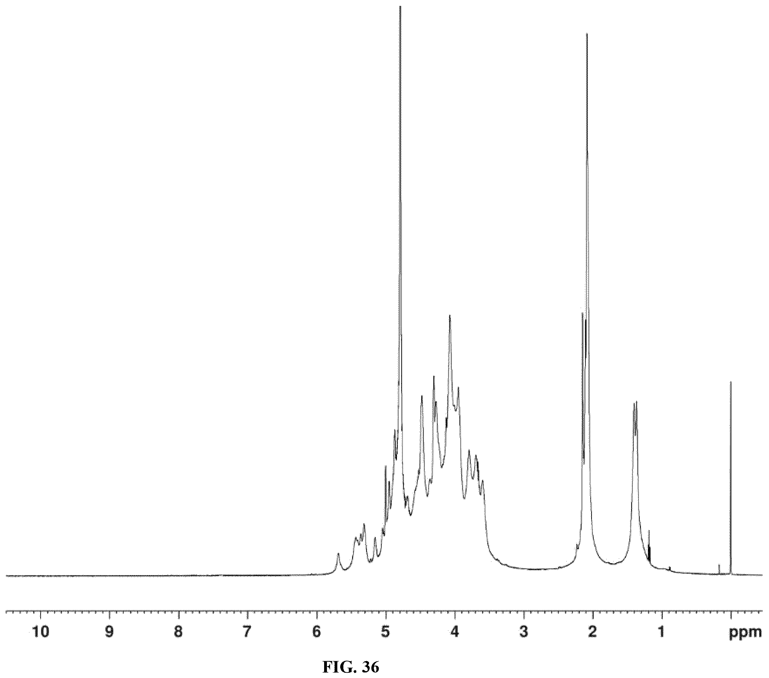

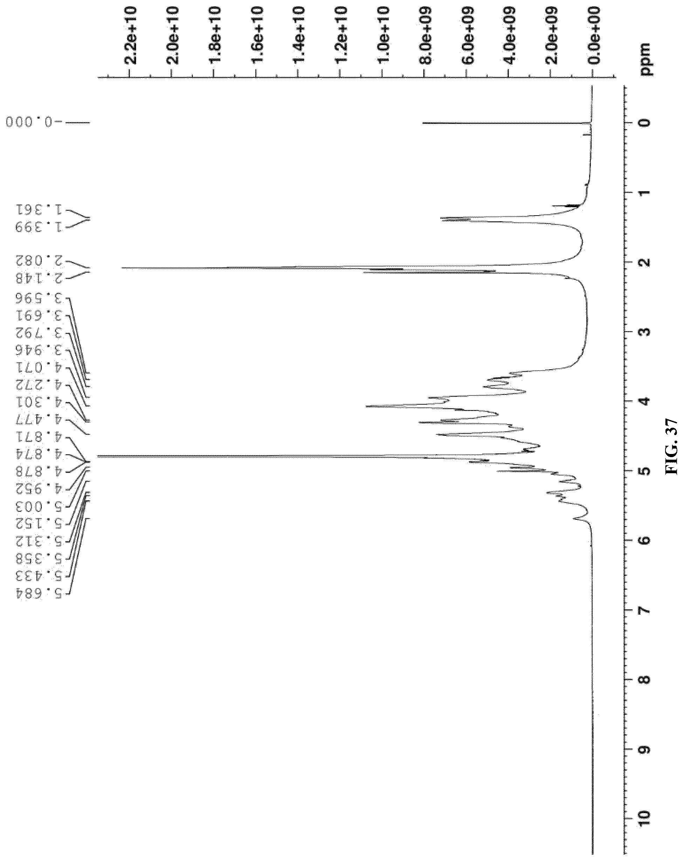

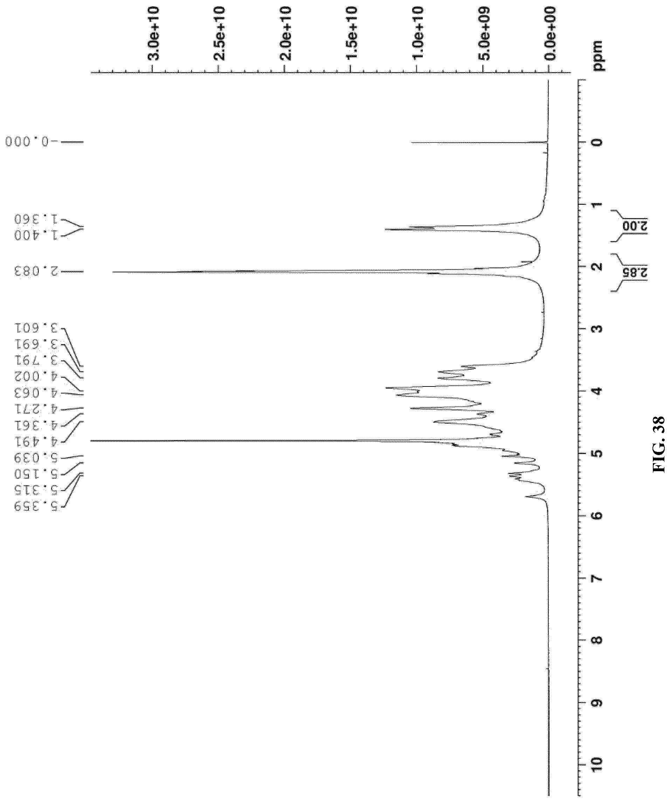

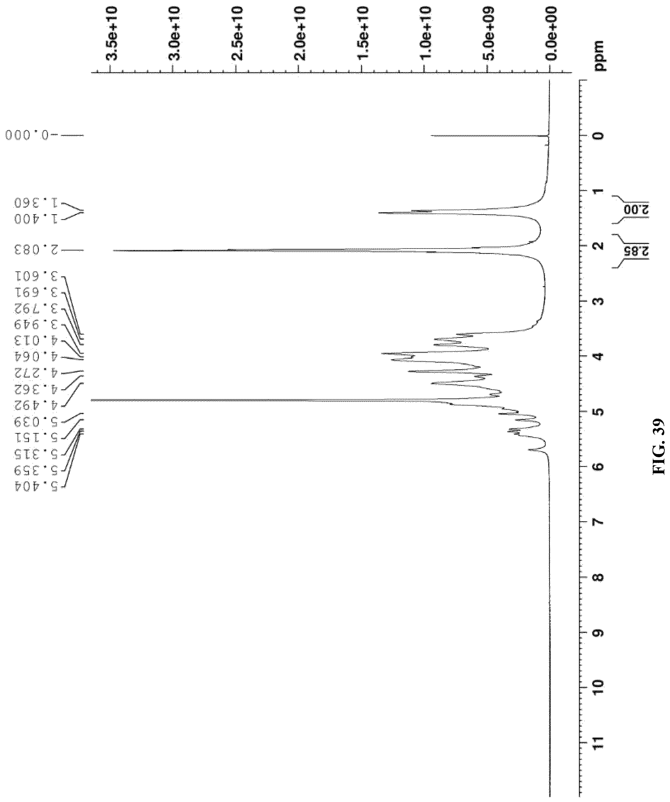

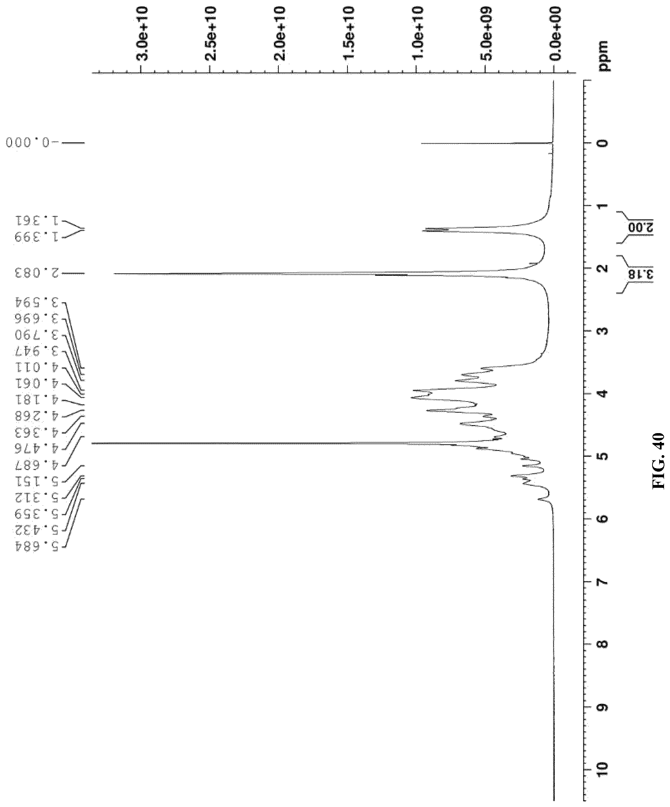

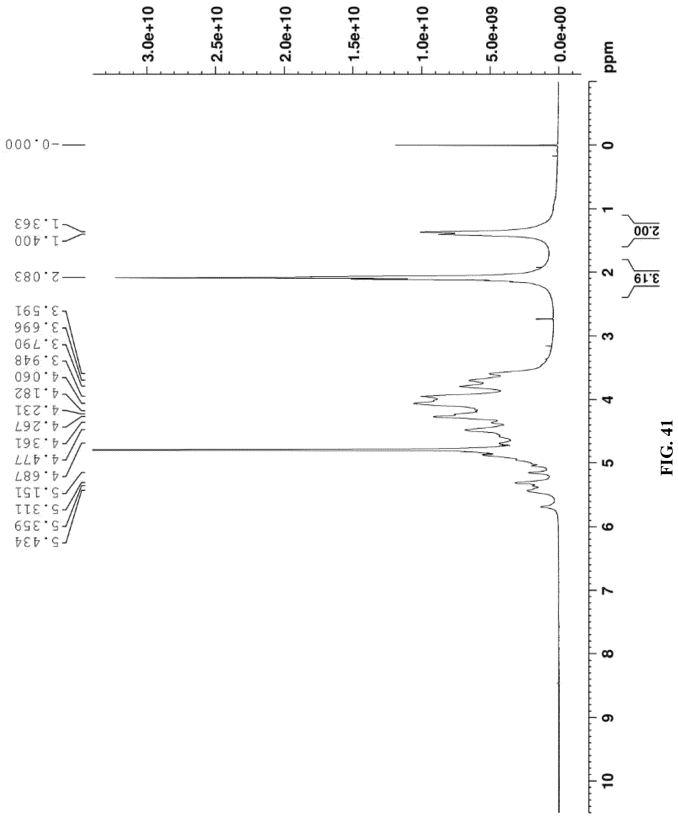

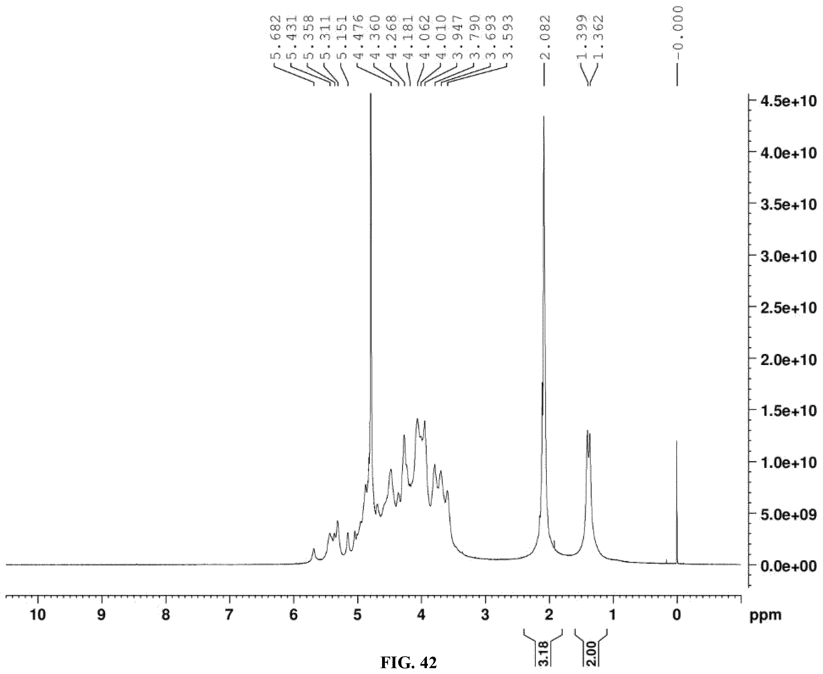

FIG. 1 shows the ¹H NMR test spectrum of the HPS; FIG. 2 shows the ¹³C NMR test spectrum of the HPS; FIG. 3 shows the heteronuclear singular quantum correlation (HSQC) test spectrum of the HPS; FIG. 4 shows the ¹H NMR spectrum of the standard solution of sample No. 400-30-2-2-8; FIG. 5 shows the ¹H NMR spectrum of the standard solution of sample No. 600-30-2-2-8; FIG. 6 shows the ¹H NMR spectrum of the standard solution of sample No. 600-90-2-2-8; FIG. 7 shows the ¹H NMR spectrum of the standard solution of sample No. 600-90-0.8-2-8; FIG. 8 shows the ¹H NMR spectrum of the standard solution of sample No. 600-90-3-2-8; FIG. 9 shows the ¹H NMR spectrum of the standard solution of sample No. 600-90-2-4-8; FIG. 10 shows the ¹H NMR spectrum of the standard solution of sample No. 600-90-2-8-8; FIG. 11 shows the ¹H NMR spectrum of the standard solution of sample No. 600-90-2-12-8; FIG. 12 shows the ¹H NMR spectrum of the standard solution of sample No. 600-90-2-16-8; FIG. 13 shows the ¹H NMR spectrum of the standard solution of sample No. 600-90-2-12-4; FIG. 14 shows the ¹H NMR spectrum of the standard solution of sample No. 600-90-2-12-8; FIG. 15 shows the ¹H NMR spectrum of the standard solution of sample No. 600-90-2-12-16; FIG. 16 shows the ¹H NMR spectrum of the standard solution of sample No. 600-90-2-12-32; FIG. 17 shows the ¹H NMR spectrum of the standard solution of sample No. 600-90-2-12-16-5; FIG. 18 shows the ¹H NMR spectrum of the standard solution of sample No. 600-90-2-12-16-10; FIG. 19 shows the ¹H NMR spectrum of the standard solution of sample No. 600-90-2-12-16-20; FIG. 20 shows the ¹H NMR spectrum of the standard solution of sample No. 600-90-2-12-16-30; FIG. 21 shows the ¹H NMR spectrum of the system suitability solution of sample No. 400-30-2-2-8; FIG. 22 shows the ¹H NMR spectrum of the system suitability solution of sample No. 600-30-2-2-8; FIG. 23 shows the ¹H NMR spectrum of the system suitability solution of sample No. 600-90-2-2-8; FIG. 24 shows the ¹H NMR spectrum of the system suitability solution of sample No. 600-90-0.8-2-8; FIG. 25 shows the ¹H NMR spectrum of the system suitability solution of sample No. 600-90-3-2-8; FIG. 26 shows the ¹H NMR spectrum of the system suitability solution of sample No. 600-90-2-4-8; FIG. 27 shows the ¹H NMR spectrum of the system suitability solution of sample No. 600-90-2-8-8; FIG. 28 shows the ¹H NMR spectrum of the system suitability solution of sample No. 600-90-2-12-8; FIG. 29 shows the ¹H NMR spectrum of the system suitability solution of sample No. 600-90-2-16-8; FIG. 30 shows the ¹H NMR spectrum of the system suitability solution of sample No. 600-90-2-12-4; FIG. 31 shows the ¹H NMR spectrum of the system suitability solution of sample No. 600-90-2-12-16; FIG. 32 shows the ¹H NMR spectrum of the system suitability solution of sample No. 600-90-2-12-32; FIG. 33 shows the ¹H NMR spectrum obtained from the 1st test of the system suitability solution in Example 3; FIG. 34 shows the ¹H NMR spectrum obtained from the 2nd test of the system suitability solution in Example 3; FIG. 35 shows the ¹H NMR spectrum obtained from the 3rd test of the system suitability solution in Example 3; FIG. 36 shows the ¹H NMR spectrum obtained from the 4th test of the system suitability solution in Example 3; FIG. 37 shows the ¹H NMR spectrum obtained from the 5th test of the system suitability solution in Example 3; FIG. 38 shows the ¹H NMR spectrum of HPS of batch 202405 in Example 3; FIG. 39 shows the ¹H NMR spectrum of HPS of batch 202404 in Example 3; FIG. 40 shows the ¹H NMR spectrum of HPS of batch 202403 in Example 3; FIG. 41 shows the ¹H NMR spectrum of HPS of batch 202402 in Example 3; and FIG. 42 shows the ¹H NMR spectrum of HPS of batch 202401 in Example 3.

### DETAILED DESCRIPTION OF THE EMBODIMENTS

First, a description of HPS is provided.

In the present disclosure, the HPS refers to a natural polysaccharide extracted from the body wall of a sea cucumber. The sea cucumber includes, but is not limited to, one or more of *Holothuria leucospilota* (Brandt), *Holothuria scabra, Stichopus japonicus, Holothuria atra,* and *Holothuria nobilis,* and are preferably *Holothuria leucospilota* (Brandt). The HPS is a fucosylated chondroitin sulfate polysaccharide. In some embodiments, the HPS has a weight-average molecular weight of 90,000 to 130,000, a polydispersity index less than or equal to 2, and is composed of monosaccharides including glucuronic acid, N-acetyl-D-galactosamine, and fucose, where a molar ratio of the glucuronic acid, the N-acetyl-D-galactosamine, and the fucose is in a range of 1 : 0.8-1.2 : 0.5-0.8; a sulfate ester group in the HPS has a mass percentage of 25% to 40%.

The HPS has a structural unit shown in Formula I: 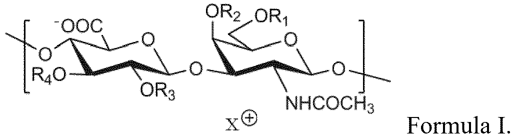 In the Formula I, R₁ is selected from the group consisting of H, SO₃⁻, and fucosyl; R₂ is selected from the group consisting of H and SO₃⁻; R₃ is selected from the group consisting of H and SO₃⁻; R₄ is selected from the group consisting of H and fucosyl; at least one of the R₁ and the R₄ is fucosyl; and X^{⊕} represents a cation.

The fucosyl for R₁ and R₄ has a structure shown in Formula I-1: 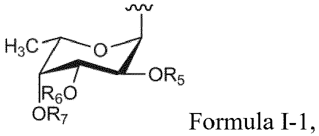 In the Formula I-1: R₅ is selected from the group consisting of H and SO₃⁻; R₆ is selected from the group consisting of H and SO₃⁻; and R₇ is selected from the group consisting of H and SO₃⁻.

In some embodiments, in the Formula I, the cation is selected from the group consisting of hydrogen ion, an alkali metal ion, an alkaline earth metal ion, an ammonium ion, and an iron ion; specifically, it may be hydrogen ion, potassium ion, sodium ion, calcium ion, ammonium ion, or iron ion; preferably, it is sodium ion. In some embodiments, under the condition that the cation is sodium ion, the sodium ion in the HPS has a mass percentage of 5% to 15%.

Evaluation results of the anticoagulant activity of HPS in mice *in vivo* indicate that HPS can significantly prolong the clotting time of activated partial thromboplastin time (APTT) in mice and rats, without adversely affecting the clotting times of prothrombin time (PT) and thrombin time (TT). These results suggest that HPS targets the endogenous coagulation pathway and does not affect the exogenous coagulation pathway.

Referring to the pharmacopoeia general chapter 1208 "heparin biological assay method", tests are conducted for the anti-Factor IIa activity, anti-Factor Xa activity, AT-III-IIa activity, and AT-III-Xa activity of HPS. The results show that at an experimental dose of 500 µg/mL, HPS displays no activity against Factor IIa, Factor Xa, or AT-III-IIa, and only shows weak activity against AT-III-Xa (with an inhibition rate of 36.7%). The activity of the rate-limiting enzyme of the endogenous coagulation pathway, "Intrinsic Factor Xase (iFXase)", is tested using the enzyme-linked immunosorbent assay (ELISA). The results indicate that the test drug HPS can significantly inhibit the endogenous coagulation pathway rate-limiting enzyme iFXase, with an IC₅₀ value of 207.5 ng/mL. The above experiments further clarify, mechanistically, that HPS can selectively inhibit the activity of the endogenous coagulation pathway rate-limiting enzyme iFXase, targeting the endogenous coagulation pathway.

The identification method of the present disclosure is described in detail below.

The present disclosure provides an identification method of an HPS by NMR spectroscopy, including the following steps:
mixing a sample to be tested, an internal standard, and deuterium oxide to obtain a test solution;
subjecting the test solution to NMR spectroscopy test, thereby obtaining an ¹H NMR spectrum; and
analyzing the ¹H NMR spectrum, and identifying whether the sample to be tested is a qualified product of the HPS based on an obtained analysis result.

In the present disclosure, a sample to be tested, an internal standard, and deuterium oxide are mixed to obtain a test solution. In some embodiments, the internal standard is sodium 3-(trimethylsilyl)propionate-2,2,3,3-d4; in some embodiments, a concentration of the sample to be tested in the test solution is not less than 20 mg/mL, preferably 20 mg/mL to 30 mg/mL; in some embodiments, a concentration of the internal standard in the test solution is 0.002% (w/v).

In the present disclosure, after obtaining the test solution, the test solution is subjected to NMR spectroscopy test, thereby obtaining an ¹H NMR spectrum. In some embodiments, the NMR spectroscopy test is conducted under conditions including: a frequency not less than 600 MHz, a pulse angle of 90°, an acquisition time not less than 2 s, preferably 2 s to 3 s, a relaxation delay not less than 12 s, preferably 12 s to 16 s, a number of scans not less than 16, preferably 16 to 32, and a spectral width covering at least a range from 10 ppm to 0 ppm.

In some embodiments of the present disclosure, an instrument mode for the NMR spectroscopy test is a pulsed Fourier transform mode; in some embodiments, the NMR spectroscopy test is conducted at a temperature of 20 °C to 30 °C; in specific examples, the NMR spectroscopy is preferably conducted at room temperature.

In the present disclosure, after obtaining the ¹H NMR spectrum, the ¹H NMR spectrum is analyzed, and whether the sample to be tested is a qualified product of the HPS is identified based on an obtained analysis result. An ¹H NMR spectrum of the qualified product of the HPS has standards including:
the ¹H NMR spectrum of the qualified product of the HPS, there are signal peaks of fucose anomeric hydrogen (denoted as Signal 1), N-acetyl methyl hydrogen (denoted as Signal 2), and fucose methyl hydrogen (denoted as Signal 3); relative to a methyl signal of the internal standard (namely the sodium 3-(trimethylsilyl)propionate-2,2,3,3-d4), a signal peak of the fucose anomeric hydrogen has a relative chemical shift of 5.68 ppm ± 0.03 ppm, a signal peak of the N-acetyl methyl hydrogen has a relative chemical shift of 2.08 ppm ± 0.03 ppm, and a signal peak of the fucose methyl hydrogen is a doublet centered at a relative chemical shift of 1.38 ppm ± 0.03 ppm;
the signal peak of the N-acetyl methyl hydrogen in the ¹H NMR spectrum has a signal-to-noise ratio not less than 2000 : 1;
in relative chemical shifts of 0.2 ppm to 0.8 ppm and 6.50 ppm to 10 ppm of the ¹H NMR spectrum, no unidentified signals greater than 8% of an intensity of the signal peak of the fucose methyl hydrogen are found; in a relative chemical shift of 3 ppm to 6 ppm of the ¹H NMR spectrum, no unidentified signals greater than 120% of the intensity of the signal peak of the fucose methyl hydrogen are found; and the intensity of the signal peak of the fucose methyl hydrogen is expressed as an average intensity of the doublet centered at the relative chemical shift of 1.38 ppm ± 0.03 ppm;
an integral area ratio value of the signal peak of the N-acetyl methyl hydrogen to the signal peak of the fucose methyl hydrogen in the ¹H NMR spectrum is in a range of 1.2 to 1.8; the signal peak of the N-acetyl methyl hydrogen is integrated in a relative chemical shift range of 1.8 ppm to 2.4 ppm, and the signal peak of the fucose methyl hydrogen is integrated in a relative chemical shift range of 1.1 ppm to 1.6 ppm.

In the present disclosure, the chemical shifts in the ¹H NMR spectrum are all relative chemical shifts to the methyl signal peak of sodium 3-(trimethylsilyl)propionate-2,2,3,3-d4, where the methyl signal peak of sodium 3-(trimethylsilyl)propionate-2,2,3,3-d4 is defined as 0.00 ppm, which will not be repeated hereafter.

In the present disclosure, if the ¹H NMR spectrum of the sample to be tested meets the above standards, the sample to be tested can be determined to be a qualified product of the HPS; otherwise, it is determined to be an unqualified product of the HPS.

In the present disclosure, the HPS is a fucosylated chondroitin sulfate, whose backbone is a copolymer composed of disaccharide units of *N*-acetyl-*D*-galactosamine and glucuronic acid. Structural differences lie only in the content and modification sites of the fucosyl and sulfate ester groups. Based on the ¹H NMR spectrum, the characteristic hydrogen signals identified in the HPS structure are the fucose anomeric hydrogen (5.0 ppm to 6.0 ppm), where the signal at 5.68 ppm ± 0.03 ppm can be clearly distinguished from other signal peaks; the signal of the acetyl methyl hydrogen in *N*-acetyl-*D*-galactosamine at 2.08 ppm ± 0.03 ppm and the signal of the fucose methyl hydrogen at 1.38 ppm ± 0.03 ppm are also clearly distinguishable from other signal peaks. Therefore, signal peaks of the fucose anomeric hydrogen, the N-acetyl methyl hydrogen, and the fucose methyl hydrogen are regarded as the characteristic peaks for identifying HPS.

Furthermore, in the chemical shift range of 2.08 ppm to 5.00 ppm of the ¹H NMR spectrum, signals arising from the HPS itself, as well as the signal from deuterium oxide, can be observed.

The following clearly and completely describes the technical solutions of the present disclosure in conjunction with the examples. Apparently, the described examples are merely some rather than all of the examples of the present disclosure. All other examples obtained by a person of ordinary skill in the art based on the examples of the present disclosure without creative efforts shall fall within the scope of the present disclosure.

### Example 1 Extraction of HPS

The body walls of *Holothuria leucospilota* (Brandt) were carefully separated from other tissues and immediately immersed in acetone, then stored at 4 °C for 24 h. Dried body wall tissue (500 g) was ground into a powder and suspended in 10 L of 0.1 mol/L sodium acetate buffer (pH=6) containing 50 g of papain. A suspension was incubated at 65 °C for 24 h. An incubation solution was then centrifuged at low temperature (4,000 rpm, 4 °C for 30 min) to obtain an enzymatic hydrolysate supernatant. A 6 mol/L aqueous hydrochloric acid solution was added to the enzymatic hydrolysate supernatant to adjust the pH value to 2.5 ± 0.5. A mixture was reacted for 2 h under stirring, followed by low-temperature centrifugation (4,000 rpm, 4 °C for 30 min) to obtain an acid hydrolysate supernatant. A 40 wt% aqueous sodium hydroxide solution was added to the acid hydrolysate supernatant to adjust the pH value to 7.0 ± 0.5. Under stirring, 1-fold volume of 95 vol% ethanol (relative to the supernatant volume) was added to a resulting reaction system. A resulting mixture was maintained at -10 °C for 24 h and then centrifuged at low temperature (4,000 rpm, 4 °C for 30 min); and a precipitate formed was collected. The precipitate was dissolved in 500 mL of distilled water, 1 L of 95 vol% ethanol was then added thereto, and a resulting mixture was maintained at -10 °C for 24 h and then centrifuged at low temperature (4,000 rpm, 4 °C for 30 min); and a precipitate formed was collected. The precipitate was washed with 500 mL of 95 vol% ethanol, and centrifuged at low temperature (4,000 rpm, 4 °C for 30 min), and a resulting solid was collected. The solid was dissolved in 500 mL of distilled water and freeze-dried to obtain 4 g of crude HPS.

The 4 g of crude HPS was loaded onto a DEAE cellulose column (10 cm × 4 cm) pre-equilibrated with 0.1 mol/L acetic acid-sodium acetate (HAc-NaAc) buffer (pH=6). The cellulose column was sequentially rinsed with 5 L of HAc-NaAc buffer (pH=6) containing 0.5 mol/L NaCl and 2 L of HAc-NaAc buffer (pH=6) containing 1 mol/L NaCl, with a flow rate in the cellulose column of 50 mL/min, and 500 mL of each fraction eluted was collected. The fractions eluted were analyzed using HPLC methods for molecular weight and molecular weight distribution. Fractions eluted of chromatographic peaks corresponding to a weight-average molecular weight between 90,000 and 130,000 were collected. 1-fold volume of 95 vol% ethanol (relative to a volume of an effluent collected) was added to the effluent, and a resulting mixture was maintained at -10 °C for 24 h and then centrifuged at low temperature (4,000 rpm, 4 °C for 30 min); and a precipitate formed was collected. The precipitate was dissolved in distilled water and filtered with an ultrafiltration membrane with a molecular weight cut-off of 10,000, thereby reaching one-half its original volume. Distilled water was then added to restore the original volume, and a resulting solution was ultra-filtered to reach one-half the original volume. Distilled water was added again to restore the original volume, and the resulting solution was concentrated to one-half the original volume. Distilled water was added to restore the original volume, and the resulting solution was concentrated to one-half the original volume. A finally obtained concentrate was collected, and freeze-dried to obtain 2.5 g of purified HPS (with sodium as the cation). Following the above procedure, 5 consecutive batch samples were prepared, designated as HPS batches 202401, 202402, 202403, 202404, and 202405.

### Example 2 Establishment of the Identification Method of HPS by NMR spectroscopy

### 1. Specificity Confirmation

The ¹H, ¹³C, and HSQC NMR spectra of HPS (Batch No.: 202401) were measured (FIG. 1 to FIG. 3) to confirm assignments of characteristic peaks of the standard sample and to establish the specificity criteria for the identification method. The HPS of the present disclosure is a fucosylated chondroitin sulfate, whose backbone is a copolymer composed of disaccharide units of *N*-acetyl-*D*-galactosamine and glucuronic acid. Structural differences lie only in the content and modification sites of the fucosyl and sulfate ester groups. Based on two-dimensional NMR spectrum, the characteristic hydrogen signals identified in the HPS structure were the fucose anomeric hydrogen (5.0-6.0 ppm), where the signal at 5.68 ppm ± 0.03 ppm could be clearly distinguished from other signal peaks; a signal of the acetyl methyl hydrogen in *N*-acetyl-*D*-galactosamine at 2.08 ppm ± 0.03 ppm and a signal of the fucose methyl hydrogen at 1.38 ppm ± 0.03 ppm were also clearly distinguished from other signal peaks. Therefore, the fucose anomeric hydrogen, the *N*-acetyl methyl hydrogen, and the fucose methyl hydrogen were selected as the characteristic peaks for identifying HPS, where the signal of the fucose methyl hydrogen was a doublet centered at 1.38 ppm ± 0.03 ppm.

### 2. Preparation of Solutions

Standard solution: the HPS identification standard sample (Batch No.: 202401) was dissolved in deuterium oxide to prepare a solution of a specific concentration, and 0.002% (w/v) sodium 3-(trimethylsilyl)propionate-2,2,3,3-d4 (TSP) was added as an internal standard.

System suitability solution: the oversulfated chondroitin sulfate reference sample (abbreviated as OCS RS) was dissolved in the standard solution to prepare a solution with a concentration of 0.3% (w/w).

The sources of reagents used for solution preparation were as follows: deuterium oxide (99.9%, Shanghai Bidepharm Co., Ltd., China); sodium 3-(trimethylsilyl)propionate-2,2,3,3-d4 (98 atom% D, J&K Scientific Ltd. Peking, China); OCS RS (Batch No.: 140789-202202, National Institutes for Food and Drug Control).

### 3. Screening of NMR Instrument Test Conditions

Instrumentation: Bruker 600 MHz AVANCE NEO (No.H03128OB/05.02/R/6852);
Bruker 400 MHz AVANCE III HD (No.2138276/ECL02.00/00311).
Software: Topspin 4.3.1.

The standard solution and the system suitability solution were used to investigate the instrument frequency, test pulse angle, acquisition time, relaxation delay, number of scans, and sample concentration. A signal-to-noise ratio of the signal peak of the *N*-acetyl methyl hydrogen (abbreviated as acetyl SN ratio) and the doublet signal intensity of the fucose methyl hydrogen (recorded as methyl peak 1 and methyl peak 2) were used as evaluation criteria. The test results are shown in Tables 1 to 2 and FIG. 4 to FIG. 32.

**Table 1 Investigation Results of Standard Solution**

| Investigation item | Sample serial number | Parameter | Acetyl SN ratio | Intensity of methyl peak 1 | Intensity of methyl peak 2 |
|---|---|---|---|---|---|
| Frequency (Hz) | 400-30-2-2-8 | 400 | 229 | 67529.56 | 73775.56 |
| | 600-30-2-2-8 | 600 | 999.05 | 4447131648 | 4511549440 |
| Pulse angle | 600-30-2-2-8 | 30 | 999.05 | 4447131648 | 4511549440 |
| | 600-90-2-2-8 | 90 | 1631.67 | 2871409152 | 2905466880 |
| Acquisition time (s) | 600-90-0.8-2-8 | 0.8 | 1587.93 | 2647890432 | 2665329664 |
| | 600-90-2-2-8 | 2 | 1631.67 | 2871409152 | 2905466880 |
| | 600-90-3-2-8 | 3 | 1711.51 | 2969261056 | 3012241408 |
| Relaxation delay (s) | 600-90-2-2-8 | 2 | 1631.67 | 2871409152 | 2905466880 |
| | 600-90-2-4-8 | 4 | 1737.49 | 3028553728 | 3075603968 |
| | 600-90-2-8-8 | 8 | 1859.81 | 3110326272 | 3153437696 |
| | 600-90-2-12-8 | 12 | 1852.45 | 3119856640 | 3175771136 |
| | 600-90-2-16-8 | 16 | 1871.76 | 3126953984 | 3174063104 |
| Number of scans (times) | 600-90-2-12-4 | 4 | 1344.33 | 1560800256 | 1585274368 |
| | 600-90-2-12-8 | 8 | 1852.45 | 3119856640 | 3175771136 |
| | 600-90-2-12-16 | 16 | 2631.61 | 6251270144 | 6362361856 |
| | 600-90-2-12-32 | 32 | 3790.85 | 12500344832 | 12730089472 |
| Sample concentration (mg/mL) | 600-90-2-12-16-5 | 5 | 737.82 | 2798198784 | 2910732288 |
| | 600-90-2-12-16-10 | 10 | 1378.37 | 4647538688 | 4769763328 |
| | 600-90-2-12-16-20 | 20 | 2631.61 | 6251270144 | 6362361856 |
| | 600-90-2-12-16-30 | 30 | 3434 | 9116123136 | 9202376704 |

In Table 1, the numbers in the sample serial number sequentially represent frequency, pulse angle, acquisition time, relaxation delay, and number of scans. For example, 400-30-2-2-8 indicates testing was conducted under conditions: a frequency of 400 MHz, a pulse angle of 30°, an acquisition time of 2 s, a relaxation delay of 2 s, and number of scans of 8. The concentration of the standard sample in the standard solution used in the frequency, pulse angle, acquisition time, relaxation delay, and number of scans tests was 20 mg/mL. In the sample concentration test, the last digit in the sample serial number represents the sample concentration value.

**Table 2 Investigation Results of system suitability solution**

| Investigation item | Sample serial number | Parameter | HPS acetyl chemical shift (ppm) | OCS RS acetyl chemical shift (ppm) |
|---|---|---|---|---|
| Frequency (Hz) | 400-30-2-2-8 | 400 | 2.0817 | 2.1476 |
| | 600-30-2-2-8 | 600 | 2.0824 | 2.148 |
| Pulse angle | 600-30-2-2-8 | 30 | 2.0824 | 2.148 |
| | 600-90-2-2-8 | 90 | 2.0823 | 2.1479 |
| Acquisition time (s) | 600-90-0.8-2-8 | 0.8 | 2.0822 | 2.1479 |
| | 600-90-2-2-8 | 2 | 2.0823 | 2.1479 |
| | 600-90-3-2-8 | 3 | 2.0822 | 2.1479 |
| Relaxation delay (s) | 600-90-2-2-8 | 2 | 2.0823 | 2.1479 |
| | 600-90-2-4-8 | 4 | 2.0823 | 2.148 |
| | 600-90-2-8-8 | 8 | 2.0823 | 2.1479 |
| | 600-90-2-12-8 | 12 | 2.0823 | 2.1479 |
| | 600-90-2-16-8 | 16 | 2.0823 | 2.148 |
| | 600-90-2-12-4 | 4 | 2.0822 | 2.1479 |
| Number of scans (times) | 600-90-2-12-8 | 8 | 2.0823 | 2.1479 |
| | 600-90-2-12-16 | 16 | 2.0823 | 2.148 |
| | 600-90-2-12-32 | 32 | 2.0823 | 2.148 |

In Table 2, the numbers in the sample serial number sequentially represent frequency, pulse angle, acquisition time, relaxation delay, and number of scans. For example, 400-30-2-2-8 indicates testing was conducted under conditions: a frequency of 400 MHz, a pulse angle of 30°, an acquisition time of 2 s, a relaxation delay of 2 s, and number of scans of 8. In the system suitability solution condition investigation, the concentration of the standard sample in the system suitability solution used was 20 mg/mL.

Based on the above test results, and considering the signal-to-noise ratio, characteristic peak intensity, and chemical shift results, along with a practical assessment of factors like sample consumption and testing time, the NMR test conditions were determined as follows: instrument mode: pulsed Fourier transform mode; frequency: not less than 600 MHz (for ¹H); temperature: 20 °C to 30 °C; pulse angle: 90°; acquisition time: not less than 2 s; relaxation delay: at least 12 s; number of scans: not less than 16; spectral width: at least 10-0 ppm.

The NMR test conditions adopted in subsequent experiments were as follows: instrument mode: pulsed Fourier transform mode; frequency: 600 MHz; pulse angle: 90°; acquisition time: 2 s; relaxation delay: 12 s; and number of scans: 16. The test temperature was room temperature.

With the established test parameters, tests were conducted using the standard solution to determine the average intensity of the doublet signal of the fucose methyl hydrogen (recorded as the average methyl peak intensity), and the integral areas of the *N*-acetyl methyl hydrogen signal peak (Signal 2) and the fucose methyl hydrogen signal peak (Signal 3) were measured in the ¹H NMR spectrum of the standard sample, and their ratio was calculated (recorded as acetyl integral/methyl integral). The integration range for Signal 2 was 1.80-2.40 ppm; the integration range for Signal 3 was 1.10-1.60 ppm. The results are shown in Table 3.

**Table 3 Test results of standard solution**

| No. | Chemical shift (ppm) | Peak intensity | Average methyl peak intensity | Peak height/average methyl peak intensity (%) | Acetyl integral/methyl integral |
|---|---|---|---|---|---|
| 1 | 0.2-0.8 | 94602240 | 6306816000 | 1.50 | 1.52 |
| 2 | 3.0-6.0 | 6962122752 | | 110.39 | |
| 3 | 6.50-10 | 84887552 | | 1.35 | |

Peak intensity in Table 3 refers to the intensity of the strongest signal peak present within 0.2-0.8 ppm, 3.0-6.0 ppm, or 6.50-10 ppm.

### 4. Suitability Requirements

Based on the experimental results above, the suitability requirements for the identification method of the HPS by NMR spectroscopy were determined:
1) Number of scans: the number of scans was adjusted until the signal-to-noise ratio of the N-acetyl methyl hydrogen signal of HPS in the standard solution reached at least 2000 : 1.
2) Chemical shift: the methyl signal of TSP (sodium 3-(trimethylsilyl)propionate-2,2,3,3-d4) was set to 0.00 ppm for all samples; the *N*-acetyl methyl hydrogen resonances of HPS and OCS RS in the system suitability solution were observed at 2.08 ppm ± 0.03 ppm and 2.14 ppm ± 0.03 ppm, respectively.
3) The baseline was plotted from 10.00 ppm to 0.00 ppm. The chemical shifts for the fucose anomeric hydrogen (Signal 1), the *N*-acetyl methyl hydrogen of *N*-acetyl-*D*-galactosamine (Signal 2), and the fucose methyl hydrogen (Signal 3) in HPS were located at 5.68 ppm ± 0.03 ppm, 2.08 ppm ± 0.03 ppm, and 1.38 ppm ± 0.03 ppm (a doublet centered at 1.38 ppm ± 0.03 ppm), respectively. Between 2.08 ppm (Signal 2) and 5.00 ppm, signals arising from HPS and the signals from D₂O (deuterium oxide, which was used as the solvent and appeared in the NMR spectrum) were observed.
4) Acceptance criteria: in the ranges of 0.20-0.80 ppm and 6.50-10.00 ppm, no unidentified signals greater than 8% of the average intensity of the Signal 3 doublet were present; in the range of 3.00-6.00 ppm, no unidentified signals greater than 120% of the average intensity of the Signal 3 doublet were present. The integral area ratio of Signal 2 to Signal 3 was in a range of 1.2 to 1.8, where the integration range for Signal 2 was 1.8-2.4 ppm and the integration range for Signal 3 was 1.1-1.6 ppm.

### Example 3 Sample Testing

### 1. Solution Preparation

Standard solution: the HPS identification standard sample (Batch No.: 202401) was dissolved in deuterium oxide and prepared as a solution with a concentration of 20 mg/mL, and 0.002% (w/v) sodium 3-(trimethylsilyl)propionate-2,2,3,3-d4 (TSP) was added as an internal standard.

System suitability solution: the OCS RS was dissolved in the standard solution to prepare a solution with a concentration of 0.3% (w/w).

Sample solutions: the HPS samples to be identified (Batch Nos. 202401, 202402, 202403, 202404, 202405) were separately dissolved in deuterium oxide and prepared as solutions with concentrations not less than 20 mg/mL, and 0.002% (w/v) TSP was added to each solution.

### 2. NMR Spectrum Measurement

A 600 MHz NMR spectrometer was used. The pulse angle was set to 90°, the acquisition time was set to 2 s, and the relaxation delay was set to 12 s; the number of scans was set to 16, the instrument mode was set to pulsed Fourier transform mode, and the ¹H NMR spectra were obtained at room temperature.

The system suitability solution was tested consecutively 5 times. The acetyl SN ratio was greater than 2,000 and the relative standard deviation (RSD) of the average fucose methyl peak intensity was 0.026%. The results are shown in Table 4. The ¹H NMR spectra obtained from these 5 tests are shown in FIG. 33 to FIG. 37.

**Table 4 System suitability test results**

| No. | Acetyl SN ratio | Average methyl peak intensity | Methyl peak chemical shift (ppm) | Acetyl chemical shift (ppm) | OCS RS acetyl chemical shift (ppm) |
|---|---|---|---|---|---|
| 1 | 3084.83 | 7165415424 | 1.3800 | 2.082 | 2.1478 |
| 2 | 3089.22 | 7161761792 | 1.3802 | 2.0821 | 2.1478 |
| 3 | 3114.57 | 7163234304 | 1.3802 | 2.082 | 2.1478 |
| 4 | 3259.23 | 7160885248 | 1.3803 | 2.082 | 2.1478 |
| 5 | 3140.55 | 7160852480 | 1.3803 | 2.082 | 2.1478 |

The test results of the sample solutions are shown in Table 5.

**Table 5 Test results for each batch sample**

| Batch | SN ratio | Chemical shift (ppm) | Peak intensity | Average methyl peak intensity | Peak height/ average methyl peak intensity (%) | Anomeric chemical shift (ppm) | Acetyl chemical shift (ppm) | Methyl peak chemical shift (ppm) | Acetyl integral/ methyl integral |
|---|---|---|---|---|---|---|---|---|---|
| 202405 | 3323.15 | 0.2-0.8 | 180850 688 | 114591 15008 | 1.58 | 5.6887 | 2.0827 | 1.3799 | 1.42 |
| | | 3.0-6.0 | 123210 09664 | | 107.52 | | | | |
| | | 6.5-10 | 130797 568 | | 1.14 | | | | |
| 202404 | 3398.19 | 0.2-0.8 | 146108 416 | 123199 61088 | 1.19 | 5.6892 | 2.0827 | 1.3799 | 1.42 |
| | | 3.0-6.0 | 134304 03072 | | 109.01 | | | | |
| | | 6.5-10 | 827719 68 | | 0.67 | | | | |
| 20240 3 | 322 7.9 1 | 0.2-0.8 | 134885 376 | 944744 4480 | 1.43 | 5.6839 | 2.0825 | 1.3804 | 1.59 |
| | | 3.0-6.0 | 103815 82336 | | 109.89 | | | | |
| | | 6.5-10 | 733143 04 | | 0.78 | | | | |
| 202402 | 3796.13 | 0.2-0.8 | 148299 776 | 943900 8768 | 1.57 | 5.6836 | 2.0828 | 1.3810 95 | 1.59 |
| | | 3.0-6.0 | 105856 45056 | | 112.15 | | | | |
| | | 6.5-10 | 135991 296 | | 1.44 | | | | |
| 202401 | 3173.72 | 0.2-0.8 | 160423 936 | 128667 89376 | 1.25 | 5.6823 | 2.0823 | 1587.5 41 | 1.59 |
| | | 3.0-6.0 | 141464 37120 | | 109.95 | | | | |
| | | 6.5-10 | 107540 480 | | 0.84 | | | | |

In Table 5, peak intensity refers to the intensity of the strongest signal peak present within 0.2-0.8 ppm, 3.0-6.0 ppm, or 6.50-10 ppm; Average methyl peak intensity refers to the average intensity of the fucose methyl proton doublet; anomeric chemical shift refers to the chemical shift of the fucose anomeric hydrogen; acetyl chemical shift refers to the chemical shift of the acetyl methyl hydrogen; methyl peak chemical shift refers to the chemical shift of the fucose methyl proton (center of the doublet); The integration range for the acetyl was 1.8-2.4 ppm; The integration range for the methyl was 1.1-1.6 ppm.

The ¹H NMR spectra of HPS of different batches are shown in FIG. 38 to FIG. 42.

Based on Table 5 and FIG. 38 to FIG. 42, it is demonstrated that the present disclosure enables effective identification of HPS, and the identification method is simple in operation, and highly specific.

The above are merely preferred embodiments of the present disclosure. It should be noted that a person of ordinary skill in the art may further make several improvements and modifications without departing from the principle of the present disclosure, but such improvements and modifications should be deemed as falling within the scope of the present disclosure.

## Claims

1. An identification method of a Haina polysaccharide (HPS) by nuclear magnetic resonance (NMR) spectroscopy, comprising the steps of:
mixing a sample to be tested, an internal standard, and deuterium oxide to obtain a test solution;
subjecting the test solution to NMR spectroscopy test, thereby obtaining a hydrogen nuclear magnetic resonance (¹H NMR) spectrum; and
analyzing the ¹H NMR spectrum, and identifying whether the sample to be tested is a qualified product of the HPS based on an obtained analysis result; wherein
the NMR spectroscopy test is conducted under conditions comprising: a frequency not less than 600 MHz, a pulse angle of 90°, an acquisition time not less than 2 seconds (s), a relaxation delay not less than 12 s, a number of scans not less than 16, and a spectral width covering at least a range from 10 ppm to 0 ppm;
a standard for the ¹H NMR spectrum of the qualified product of the HPS comprises:
in the ¹H NMR spectrum of the qualified product of the HPS, there are signal peaks of fucose anomeric hydrogen, N-acetyl methyl hydrogen, and fucose methyl hydrogen;
relative to a methyl signal of the internal standard, a signal peak of the fucose anomeric hydrogen has a relative chemical shift of 5.68 ppm ± 0.03 ppm, a signal peak of the N-acetyl methyl hydrogen has a relative chemical shift of 2.08 ppm ± 0.03 ppm, and a signal peak of the fucose methyl hydrogen is a doublet centered at a relative chemical shift of 1.38 ppm ± 0.03 ppm;
the signal peak of the N-acetyl methyl hydrogen in the ¹H NMR spectrum has a signal-to-noise ratio not less than 2000 : 1;
in relative chemical shifts of 0.2 ppm to 0.8 ppm and 6.5 ppm to 10 ppm of the ¹H NMR spectrum, no unidentified signals greater than 8% of an intensity of the signal peak of the fucose methyl hydrogen are found; in a relative chemical shift of 3 ppm to 6 ppm of the ¹H NMR spectrum, no unidentified signals greater than 120% of the intensity of the signal peak of the fucose methyl hydrogen are found; and the intensity of the signal peak of the fucose methyl hydrogen is expressed as an average intensity of the doublet centered at the relative chemical shift of 1.38 ppm ± 0.03 ppm; and
an integral area ratio value of the signal peak of the N-acetyl methyl hydrogen to the signal peak of the fucose methyl hydrogen in the ¹H NMR spectrum is in a range of 1.2 to 1.8, wherein the signal peak of the N-acetyl methyl hydrogen is integrated in a relative chemical shift range of 1.8 ppm to 2.4 ppm, and the signal peak of the fucose methyl hydrogen is integrated in a relative chemical shift range of 1.1 ppm to 1.6 ppm; and
the qualified product of the HPS is a fucosylated chondroitin sulfate polysaccharide; and the HPS has a weight-average molecular weight of 90,000 to 130,000, and is composed of monosaccharides comprising glucuronic acid, *N*-acetyl-*D*-galactosamine, and fucose, wherein a molar ratio of the glucuronic acid, the *N*-acetyl-*D*-galactosamine, and the fucose is in a range of 1 : 0.8-1.2 : 0.5-0.8; and a sulfate ester group in the HPS has a mass percentage of 25% to 40%.

2. The identification method as claimed in claim 1, wherein the sample to be tested in the test solution has a concentration not less than 20 mg/mL.

3. The identification method as claimed in claim 2, wherein the sample to be tested in the test solution has the concentration of 20 mg/mL to 30 mg/mL.

4. The identification method as claimed in claim 1 or 2, wherein the internal standard is sodium 3-(trimethylsilyl)propionate-2,2,3,3-d4.

5. The identification method as claimed in claim 1 or 2, wherein the internal standard in the test solution has a concentration of 0.002% weight/volume (w/v).

6. The identification method as claimed in claim 1, wherein the NMR spectroscopy test is conducted with the acquisition time of 2 s to 3 s.

7. The identification method as claimed in claim 1, wherein the NMR spectroscopy test is conducted with the relaxation delay of 12 s to 16 s.

8. The identification method as claimed in claim 1, wherein the NMR spectroscopy test is conducted with the number of scans of 16 to 32.

9. The identification method as claimed in claim 1, wherein the NMR spectroscopy test is conducted using an instrument mode of a pulsed Fourier transform mode.

10. The identification method as claimed in claim 1, wherein the NMR spectroscopy test is conducted at a temperature of 20 °C to 30 °C.

11. The identification method as claimed in claim 10, wherein the NMR spectroscopy test is conducted at room temperature.
